# EUROPEAN PATENT SPECIFICATION

(11) **EP 1 689 746 B1**
(45) Date of publication and mention of the grant of the patent: **09.01.2019**
(21) Application number: 04811266.8
(22) Date of filing: 12.11.2004
(51) Int. Cl.: C07D 471/00, C07D 491/00, C07D 498/00, C07D 513/00, C07D 515/00, C08K 3/20, C08K 5/34, C08K 5/48, C08K 9/00, C08L 25/00, C09B 67/22, C09B 67/04, C09B 67/52

(54) **A PROCESS FOR AQUEOUS MILLING OF QUINACRIDONE PIGMENTS**
VERFAHREN ZUM WÄSSRIGEN VERMAHLEN VON CHINACRIDONPIGMENTEN
PROCÉDÉ DE BROYAGE AQUEUX DE PIGMENTS QUINACRIDONE

(30) Priority: 13.11.2003 US 519842 P
(43) Date of publication of application: 16.08.2006
(73) Proprietor: BASF SE, 67056 Ludwigshafen am Rhein (DE)
(72) Inventor: HE, Yingxia, Wilmington, DE 19898 (US); CAMPBELL, Colin Dennis, Claymont, DE 19703 (US); SCHILLING, Gordian, 79771 Klettgau (DE); CARTER, Rhonda, Bear, DE 19701 (US)
(74) Representative: Herzog, Fiesser & Partner Patentanwälte PartG mbB
(86) International application number: PCT/US2004/038492
(87) International publication number: WO 2005/049735

(56) References cited:
- WO-A1-99/61534
- US-A- 4 293 475
- US-A- 4 713 411
- US-A- 4 857 646
- US-A- 5 281 269
- US-A- 5 383 966
- US-A1- 2001 042 492
- US-A1- 2002 075 369
- US-A1- 2002 161 232
- DATABASE WPI Week 200351 Thomson Scientific, London, GB; AN 2003-536902 & JP 2003 147243 A (RICOH KK) 21 May 2003 (2003-05-21)
- DATABASE WPI Week 200008 Thomson Scientific, London, GB; AN 2000-091060 & JP H11 323232 A (TOYO INK MFG CO LTD) 26 November 1999 (1999-11-26)

## Description

The present invention is directed to an aqueous process for reducing particle size of organic pigments by milling the crude pigment in the presence of styrene copolymer dispersants.

It is well known in the art that organic pigments, such as quinacridones, as synthesized, are generally unsuitable for use as pigments and must be further processed to develop the requisite pigmentary properties such as particle size, particle shape, polymorphic phase, and tinctorial strength.

In order to obtain the color properties required for a particular application, the pigment crude must be converted to a pigmentary grade with a proper tint strength, transparency or opacity for a particular application. The effectiveness of a given pigment type in imparting color is dependent upon it's particle size in dispersion. Thus, color strength, transparency and opacity are all properties that are highly dependant on particle size. Consequently, crude organic pigments undergo one or more finishing or conditioning steps that require particle size reduction. See, for example R.B. McKay, "Control of the Application Performance of Classical Organic Pigments" in JOCCA, 89-93.

For example, quinacridones can be synthesised from a peroxide process, as described in US patent 5,840,901. The obtained pigment is called a pigment crude because the pigment does not have the color property needed for various applications. Further size reduction is necessary. There are many traditional ways of achieving proper pigment particle size including dry milling processes, for example, those published in US patents 2,402,167, 3,030,370 and 5,084,100. These references disclose pigment particle size reduction by milling dry pigment powder in the presence of large amounts of inorganic salt and metal nails and/or balls. In this process, pigment crude is first milled to an almost amorphous phase, followed by a re-growth process in order to achieve the proper crystal size. This milling of pigment crude followed by the re-crystallization makes this finishing process complicated and costly. Another traditional pigment finishing process is acid drowning, as described in, for example, US patents 3,326,918, 3,607,336 and 4,455,173. In this process, crude organic pigment is dissolved in a strong acid such as sulphuric acid, often in the presence of polar organic solvent. Then the pigment is drowned out and re-crystallized into an appropriate particle size designed for a particular application. In both processes, the initially synthesized pigment crystal is more or less destroyed then rebuilt, adding significant cost to the final product. Further, the dry milling process requires the presence of inorganic salts and re-crystallization in strong acid, generating significant environmental waste. The dissolution process similarly generates large quantities of waste acid.

Milling methods are known for improving properties of various organic pigments. Some examples are in US 6,210,474, US 4,597,794, US 5,231,131, and US 5, 530,043. However, the milled pigment in these patents is not the crude organic pigment but a finished pigment. Additionally, the dispersing milling agent is not a styrene copolymer.

US 6,410,619 describes a method for dispersing crude organic pigments using pure acrylic copolymer dispersants. Styrene copolymer is disclosed in EP 496 149 in making aqueous emulsions for use in graphics. JP 55089366 and US 4,293,475 disclose styrene copolymers for making pigment dispersions. Patents WO9905575, EP 636,942 and US 5,432,036 disclose styrenic polymer based resins for making toner compositions for dry electro-photoconductive imaging. US 2,816,115 describes the use of a hydrolysed styrene-maleic anhydride copolymer for dispersing phthalocyanine pigments. US 6,056,814 describes the use of a styrene copolymer for dry milling a crude organic pigment.

EP 1146090 A2 discloses a process for the preparation of a pigment dispersion, the process comprising a pigment surface treatment step and a dispersion step.

US 4293475 A describes a process in which phthalocyanine was milled together with a water-soluble styrene-acrylic acid-co-(ter)polymer.

US 4713411 A discloses a pigment composition comprising 75 to 95 wt.-% of an organic pigment and a polymer comprising styrene which is prepared by forming an aqueous solution followed by dispersion of the pigment by means of a desirable disperser, e.g. a ball mill.

US 5383966 A relates to a process for conditioning quinacridone pigments by (a) preparing an aqueous slurry of a crude quinacridone; (b) thoroughly mixing the aqueous slurry with about 0.3 to about 2.9 parts by weight, relative to the quinacridone, of at least one C1-C12 alkyl ester of a C7-C12 aromatic carboxylic acid at a temperature of between about 70°C and about 200°C.; (c) hydrolyzing the alkyl ester used in step (b); and (d) collecting the quinacridone pigment.

There is a need for a process that reduces particle size but does not destroy the crystal structure of the pigment and that can be used for both particle size reduction of isomorphous crystal phases, preferably beta and gamma crystal phases of quinacridones. There is also a need for an environmentally sound process that does not produce large amounts of waste in the milling or recrystallization. Additionally there is a need for a process which allows the reduction in particle size at high pigment loadings thus increasing the manufacturing throughput. Further, there is need for a process that does not require the crude pigment to be dried, a considerable energy cost savings, before reducing particle size.

None of the above references disclose the use of a styrene copolymer dispersant as an aqueous milling agent for the purposes of reducing particle size to the nanometer range to improve the optical properties of a crude organic pigment.

The present invention relates to an aqueous particle size reduction process of quinacridone pigments using medium to high molecular weight styrene copolymer as dispersant. The products obtained optionally may be ripened under basic or acid conditions to re-grow crystals in the presence of the styrene copolymer dispersant. Thus the inventive process avoids the need for finishing and milling processes requiring the use of strong acids and/or milling with inorganic salts.

The aqueous organic pigment particle size reduction process disclosed in this invention is an economic process in comparison with known methods. This process is the first in the art to use styrene copolymer dispersants for aqueous particle size reduction of crude organic pigment to the nanometer range. The products obtained, with or without further ripening, have excellent application properties in coatings, films, plastics and inks. The product obtained also has exceptional dispersibility and light stability. This simple milling process can be used to produce transparent or semi-transparent pigment products in combination with milling, and particle size re-growth process suitable for production of high performance opaque pigments.

The isolated milled organic pigment or pigment mixtures is preferably re-grown under basic conditions to form an opaque pigment.

The pigment of the invention may be isolated and dried in pure form, in which case it is readily dispersible thereafter in plastics, paints and printing inks using, for example, a ball mill or bead mill. As a moist presscake, it can also be used directly to prepare pigment dispersions for inks, coatings and cosmetics.

The pigments made by the process of the invention (with optional further additives) can also be added to a polymer in the form of a masterbatch ("concentrate") which contains the components in a concentration of, for example, about 1 % to about 40% and preferably 2 % to about 20 % by weight incorporated in a polymer,

Dispersions of the pigments of the invention are also ideally suited as concentrates for preparing printing inks that have excellent applications properties, especially attractive colouristics with high colour strength.

The materials containing the isolated pigments made by the process of the invention described herein can be used for the production of moldings, rotomolded articles, injection molded articles, blow molded articles, films, tapes, mono-filaments, fibers, nonwovens, profiles, adhesives or putties, surface coatings and the like.

All weights unless otherwise stated are based on the total weight of the composition or mixture. The weight percent of the water-soluble styrene copolymer dispersant (ii), and the optional additive (iv) are based on the dry weight of the crude organic pigments.

The instant process for particle size reduction of organic pigments to improve optical properties of said pigments comprises
(a) milling a mixture comprising:
   i.) about 10-60 wt. % one or more crude organic pigments,
   ii.) about 0.1-25 wt. % of a water-soluble styrene copolymer dispersant based on the dry weight of the crude organic pigment,
   iii.) about 0.1-1.0 wt. % of defoamer,
   iv.) optionally about 0.1-5.0 wt. % of an additive based on the dry weight of the crude organic pigment, and
   v.) greater than about 10 wt. % of water; wherein the weight percent of components i.), iii.), and v.) are based on the total weight of the mixture, and wherein by continuously circulating the mixture through a milling media a particle size range of 30 to 300 nm of the milled pigment or milled pigment mixture is obtained
(b) isolating the milled organic pigment.

Preferably, the mixture comprises
i.) about 10-45 wt. % one or more crude organic pigments,
ii.) about 1.0-20 wt. % of a water-soluble styrene copolymer dispersant based on the dry weight of the crude organic pigment,
iii.) about 0.1 -1.0 wt. % of defoamer,
iv.) optionally about 0.1-5.0 wt. % of an additive based on the dry weight of the crude organic pigment, and
v.) greater than about 10 wt. % of water; wherein the weight percent of components i.), iii.) and v.) are based on the total weight of the mixture.

More preferably, the mixture comprises
i.) about 15-35 wt. % one or more crude organic pigments,
ii.) about 5-10 wt. % of a water-soluble styrene copolymer dispersant based on the dry weight of the crude organic pigment,
iii.) about 0.1-1.0 wt. % of defoamer,
iv.) optionally about 0.5-5.0 wt. % of an additive based on the dry weight of the crude organic pigment, and
v.) greater than about 30 wt. % of water; wherein the weight percent of components i.), iii.) and v.) are based on the total weight of the mixture.

In this process, the pigment suspension is continuously circulated through the milling media, such as zirconium oxide beads in a mill such as a Dyno-mill or Netzsch-mill. Pigment primary particle size distribution is reduced to about 30-300 nm depending on the milling time and size of milling media. Preferably the pigment primary particle size distribution is reduced to about 40-200 nm. Optionally, the re-growth process may be followed in order to achieve the opacity needed for a particular application. The products obtained from this invention process can be transparent, semi-transparent or opaque, with excellent properties of color strength, rheology, heat stability and weather durability in paint, plastics or ink applications.

Pigment crude used in this invention includes perylenes, quinacridones, phthalocyanines, isoindolines, dioxazines, triphendioxazines, 1,4-diketopyrrolopyrroles, anthrapyrimidines, anthranthrones, flavanthrones, indanthrones, perinones, pyranthrones, thioindigos, 4,4'-diamino-1,1-dianthraquinonyl, and azo compounds, as well as substituted derivatives thereof. Mixtures, including solid solutions, may also be prepared. Preferred organic pigments are the high performance pigments such as perylene, quinacridone, phthalocyanine, isoindoline, 1,4-diketopyrrolopyrroles and dioxazine pigments. Especially preferred pigments are quinacridones, perylenes and 1,4-diketopyrrolopyrroles.

### Crude pigment

The crude pigment or pigment mixtures are generally those lacking in properties required for a colorant because of inferior color development and having a particle diameter size range of about 0.2 to 40 µm, preferably 0.3 to 4 µm, most preferably about 1.0-3.0 µm. Alternatively, in the instant invention, commercially available pigments composed of pigment particles with a particle diameter size range of about 0.3 to 0.5 µm may be used as the raw materials to be milled.

In particular, the substituted and un-substituted quinacridones of formula (A) in beta and gamma crystal phases are especially preferred. 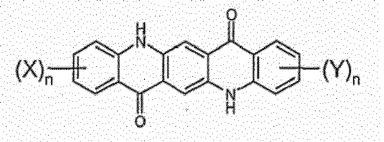 (A), or salt thereof
in which X and Y, independently of one another, are hydrogen, halogen, -OH, -NO₂, -CF₃, an C₁-C₄alkyl group, a substituted C₁-C₄alkyl group, a C₁-C₄alkoxy group, a substituted C₁-C₄alkoₕy group, a phenyl group, a cyclohexyl group, a phenoxy group, -COOH, a -COO-C₁-C₄alkyl group, -SO₃H, a phenylamino group, a benzamino group, -N(CH₃)₂, -SO₂NH₂, - SO₂N(CH₃)₂, a pyridino group, -CONH₂ or -CON(CH₃)₂, especially H, F, Cl, Br, I, C₁-C₄alkyl, or C₁-C₄alkoxy, and
n is 0, 1, or 2, especially 0, or 1,
C₁-C₄alkoxy group are defined as having up to 4 carbon atoms and is a branched or unbranched radical, for example methoxy, ethoxy, propoxy, isopropoxy, and n-butoxy.
C₁-C₄alkyl group is difined as having up to 4 carbon atoms and is a a branched or unbranched radical, for example methyl, ethyl, propyl, isopropyl, and n-butyl.

Further preferred quinacridones, such as, for example, quinacridone, 2,9-dichloroquinacridone, 3,10-dichloroquinacridone, 4,11-dichloroquinacridone, 2,3,9,10-tetrachloroquinacridone, 2,4,9,11-tetrachloroquinacridone, 2,9-difluoroquinacridone, 2,9-dibromoquinacridone, 2,9-dimethylquinacridone, 3,10-dimethylquinacridone, 4,11-dimethylquinacridone, 2,4,9,11-tetramethylquinacridone, 2,9-,di(tert-butyl)quinaceidone, 2,9-dihydroxylquinacridone, 2,9-di(trifluoromethyl)quinacridone, 2,9-dimethoxyquinacridone, 2,9-diethoxyquinacridone, 2,4,9,11-tetramethoxyquinacridone, 2,9-dicarboxylquinacridone, 2,9-dichlorohexylquinacridone, 2,9-diphenylquinacridone, 2,9-di(dimethylamino)quinacridone, 2,9-di(dimethylaminosulfo)quinacridone, 2,9-di(dimethylaminocarbonyl)quinacridone, 3,10-dinitroquinacridone, 2,9-dimethyl-4,11-dichloroquinacridone, 2,9-dimethyl-4,11-dicarboxyquinacridone, and 2,9-dipyridinoquinacridone. Quinacridones can exist in several polymorphic phases (e. g. alpha, beta, gamma phase). All are encompassed.

The most preferred quinacridone of formula (A) is a beta or gamma unsubstituted quinacridone or 2,9-dichloroquinacridone.

### Copolymer Dispersant

As copolymer dispersant for the aqueous milling of the crude pigment, it is preferred to use a copolymer consisting of hydrophilic and hydrophobic functional groups. The former portion can be ionizable and can form ammonium or alkali metal salts. The chemical structure of a typical styrene copolymer dispersant is represented below in formula (B): 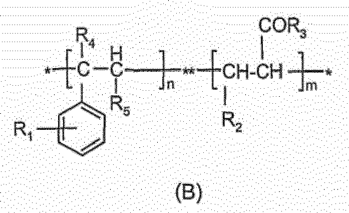 where R₁ is H, C₁C₄ alkyl, fluoro, chloro, bromo, iodo, nitro, or amino groups, C₁-C₁₈ alkylene, or C₁-C₄ alkoxy groups. R₂ is H and C₁-C₄ alkyl group, R₃ is hydroxyl or C₁-C₄ alkoxy group, and R₄ and R₅ are, independently of one another, H, or C₁-C₄ alkyl group. 100 > n > 1. 100 > m > 1. The styrene monomer unit percent in the copolymer can be in the range of about 10 - 90 mol %, although It is preferred that the styrene or styrene derivative monomer makes up greater than about 50 mol % of the hydrophobic monomer unit of the copolymer in formula (B).

The monomer unit or monomer units are defined as the monomer or monomers used in forming the final styrene copolymer.

The preferred styrene copolymer dispersant of the instant invention has a ratio of styrene to (meth)acrylic monomer units that is equal to or greater than about 2 to 1 respectively.

The copolymer is preferably a random, block or graft copolymer.

The hydrophilic portion of the copolymer is made from suitable unsaturated acid monomer units including acrylic acid, methacrylic acid, or hydrolysed esters of acrylic or methacrylic acid. Examples of suitable unsaturated acid monomer units include, (meth)acrylic acid and their lower alkyl esters such as methyl methacrylate, ethyl methacrylate, ethyl acrylate, maleic acid, fumaric acid, itaconic acid, citraconic acid, cinnamic acid, crotonic acid, and the like, and combinations thereof. The unsaturated acid monomer unit can also be in the form of a carboxylate salt with a suitable cation including sodium, potassium, and ammonium.

(Meth)acrylic acid refers to acrylic and methacrylic acid derivatives.

It is preferred that the styrene copolymer dispersant comprises a maximum of about 49 mol % of a unsaturated acid monomer unit. It is especially preferred that the unsaturated acid monomer unit is (meth)acrylic acid, a (meth)acrylic acid derivative or a carboxylate salt thereof.

Monomers which form the hydrophobic portion of the copolymers can be selected from styrene and styrene derivatives, such as C₁-C₄ alkyl or substituted styrene, vinyltoluene, alpha-methylstyrene, o-, p-, and m-chloromethyl styrene, styrene substituted with fluoro, chloro, bromo, iodo, nitro, or amino groups, butadiene, or octadecene.

The styrene copolymers used in this invention are medium to high molecular weight copolymers, with an average molecular weight in the range of about 5,000 to 20,000. Preferably the molecular weight of copolymers range from about 9,000 to 17,000.

The preferred styrenic copolymers used in this invention are styrene acrylic copolymer, which are commercially available from many specialty chemical manufacturers, such as SC Johnson Polymer (1525 Howe St.; Racine, Wis. 53403; USA). Examples of products can be all Joncryl type of styrene copolymers, including solid state product and their solutions, such as ammonium and sodium salts. Molecular weight for these styrene copolymers range from about 8,500 - 16,500. The ratio of styrene to acrylic monomer units is equal to or greater than about 2 to 1 respectively.

The styrene copolymer makes up about 0.1-20 wt. % of the dry pigment weight in wet milling mixture, preferably about 1.0-20 % wt. % and more preferably about 5 - 10% wt. % of the pigment.

The wet-milling mixture contains about 10-60 wt. % of organic pigment in the total wet milling mixture, preferably about 15-35 wt. %.

The styrene copolymer can be added before and during milling to control the viscosity of the crude pigment milling mixture.

The styrene copolymer is preferably soluble in the aqueous milling media.

### Milling

Aqueous milling is carried out using known wet-milling methods. Although the particular milling apparatus is generally not critical, suitable mills include horizontal mills, for example, Dyno-mill, Eiger mills, Netzsch mills, and Super mills. Additional vertical mills, ball mills, attritors, vibratory mills, and the like containing various grinding media are suitable. Suitable grinding media include salt, sand, glass beads, ceramic beads and alumina, or metal beads. Regardless of the type of mill used, the crude pigment, the styrenic copolymer and the other optional components are milled until the desired particle size is reached. Milling temperature depends on the size of the mill, and the quantity of crude pigment being milled but is generally carried out at a temperature of 0°C to about 60°C. Preferably the process milling temperature is 15°C to about 60°C. Optionally, cooling with water can control the temperature.

In the instant invention, the average particle diameter of the resulting aqueously milled pigment is about 30-300 nm, preferably 40-200 nm.

### Time for grinding

Particle size reduction time may vary from thirty minutes to twelve hours depending upon the particle size needed for a particular application and the particular crude pigment being wet-milled. In the case of beta-quinacridone crude, the milled product maintained beta-crystal phase after 12 hours of aqueous milling. For gamma quinacridone, gamma-II crystal phase completely converted to gamma-I phase after the crude was milled for five hours. The highest tint strength was achieved within five hours milling time. Extension of milling time allowed the pigment to develop transparency, but tint strength stayed constant, or changed only slightly.

### Grinding media

Grinding media is generally loaded to about 75%-85% of chamber space. The milling media, consists of beads composed of materials such as zirconium oxide, glass, borosilicate, metal, alumina and polymeric beads for example, those described in US 5,902,711, and US 5,478,705.

### Milling liguid

Suitable milling liquid is water, and can include less than 5 wt. % of polar organic solvent, such as dimethylformamide, dimethylacetamide, dimethylsulfoxide, lower aliphatic alcohols such as methanol, ethers Including tetrahydrofuran and dioxane, and alkylene glycols and triols such as ethylene glycol and glycerol.

The milling liquid is made up of greater than about 10 wt. % water, preferably greater than about 20 wt. % water, and most preferably greater than 30 wt. % water.

### Milling additives

Colored additives, such as organic pigment derivatives, or uncoloured additives, such as polymers, can also optionally be added to the milling mixture during the milling process.

The crude pigment may be optionally wet-milled with other additives such as surface modification reagents, rheology improving agents, texture improving agents, defoamers, wetting agents, particle growth inhibitors, crystal phase directors and antiflocculants.

Surface modifying reagents, rheology improving agents and texture improving agents may include quinacridone monosulfonic acid or quinacridone monosulfonic acid aluminum salt or 3,6-dimethylpyrazol-1-methyl quinacridone, Other suitable texture improving agents are, in particular, fatty acids of not less than 18 carbon atoms, for example stearic or behenic acid or their amides or metal salts thereof, preferably sodium or ammonium salts, as well as plasticizers, waxes, resin acids such as abietic acid or metal salts thereof, colophonium, alkyl phenols or aliphatic alcohols such as stearyl alcohol or vicinal diols such as dodecane-1,2-diol. The additives may be added directly to the milling slurry or at the same time as the crude pigment. The additive or additives may optionally be added at about 0.5-20.0 wt. %. Perferably the additive or additives are optionally added at about 1.0-5.0 wt. %.

### Defoamer

Defoamers can be used optionally in the inventive wet-milling process. Acetylenic based defoamers are preferred. The defoamer may be added before and/or during milling for foam control.

### Isolation of wet-milled crude pigment

After milling, the pigment may be separated from the milling mixture by one or more isolation methods known in the art. Filtration, followed by washing to remove residual salts and solvent, is the preferred separation method. Other collection methods known in the art, such as tray drying, spray drying, spin flash drying, lyophilization, centrifugation, or simple decantation are also suitable isolation methods. Such methods can be used individually or in combination.

In another embodiment, the instant invention is also directed to a method or use of 2,9-dichloro-quinacridone as a crystal phase inhibitor during the beta-quinacridone or gamma-quinacridone crude pigment particle size reduction processes in the presence of a water- soluble styrene copolymer dispersant.

Quinacridone (QA) is known to exist in three crystal phases. The alpha phase, described in U.S. Patent No. 2,844,484 and gamma phase described in U.S. Patent Nos. 2,844,581 and 2,969,366 forms are a bluish red color. The beta form described in U.S. Patent Nos. 2,844,485 and 4,857,646 is violet. The alpha quinacridone crystal form is not commercially valuable because it is not heat stable. See W. Herbst and K. Hunger, "Industrial Organic Pigments", VCH Publishers, Inc., 1997, page 464.

It is well known in the art that organic pigments, such as quinacridones, as synthesized, are generally unsuitable for use as pigments and must be further processed to develop the requisite pigmentary properties such as particle size, particle shape, polymorphic phase, and tinctorial strength.

In order to obtain the color properties required for a particular application, the pigment crude must be converted to a pigmentary grade with a proper tint strength, transparency or opacity for a particular application. The effectiveness of a given pigment type in imparting color is dependent upon it's particle size in dispersion. Thus, color strength, transparency and opacity are all properties that are highly dependant on particle size. Consequently, crude organic pigments undergo one or more finishing or conditioning steps that require particle size reduction. See, for example R.B. McKay, "Control of the Application Performance of Classical Organic Pigments" in JOCCA, 89-93.

Thus, the crude beta- or gamma-quinacridone usually undergoes a particle size reduction process. During this particle reduction of the beta or gamma quinacridone, the beta or gamma will tend to convert to the alpha crystal form depending on the milling conditions without a crystal phase inhibitor. As mixing of the alpha-quinacridone with either the beta or gamma phase changes product color shade and decreases heat stability of the final finished pigment, inhibition of this conversion during milling is to be avoided.

EP 517662 and US 5281269 describe an aqueous milling process of modifying beta-quinacridone (QA) with base and phase-transfer catalyst.

EP 1020497 describes the color property of the mixed crystal phase pigment with 2,9-dichloroquinacridone.

EP 799863 describes the preparation of beta-phase quinacridone by conversion of alpha-phase quinacridone.

EP 517663 and EP 517662 describe a process of preparing magenta colour beta-1 form quinacridone pigment by either dry milling of beta-quinacridone crude, or milling of beta-quinacridone crude in the presence of water and alcohol.

EP 305328 describes a new magenta color beta-quinacridone that has average particle size over 0.1 microns.

However, none of the above references disclose the use of 2,9-dichloroquinacridone as an alpha-quinacridone crystal phase inhibitor in beta quinacridone particle size reduction process.

Surprisingly, it has been discovered that the beta-quinacridone crystal phase can be preserved during particle size reduction by the addition of 2,9-dichloroquinacridone during the finishing process. The beta-quinacridone product obtained from this process has blue shade violet color that is not achievable when alpha-quinacridone exists in the product.

Furthermore, it has also been discovered that the same crystal phase inhibitor, 2,9-dichloroquinacridone can also be used with the gamma-quinacridone during the particle size reduction to prevent the formation of alpha-quinacridone. Gamma-quinacridone has red color that can be shifted to yellow or bluer shade. Particle size reduction shifts the color to a bluer shade with/without alpha-quinacridone. Beta-quinacridone develops violet color as particle size is reduced. Without alpha-quinacridone, the beta shifts to a bluer violet shade. Thus, a saturated violet color product for beta and red color for gamma is produced with better pigment properties for coatings, plastics and ink applications is achieved for both beta-quinacridone and gamma-quinacridone by milling in the presence of 2,9-dichoroquinacridone as a crystal phase inhibitor.

The desired pigmentary particle size for the purposes of the invention will vary depending upon the final application. Pigment primary particle size distribution is generally reduced to about 30-300 nm depending on the milling time and size of milling media. Preferably the pigment primary particle size distribution is reduced to about 40-200 nm. Optionally, a re-growth process may be followed in order to achieve the opacity needed for a particular application. The products obtained from this invention process can be transparent, semi-transparent or opaque.

The 2,9-dichloroquinacridone may be a crude pigment or a finished pigment.

The structure of 2,9-dichloroquinacridone used in this invention is described in formula (A). 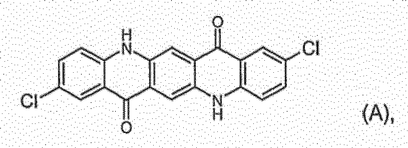 (A), or salt thereof

Beta-quinacridone or gamma-quinacridone crude pigment used in this invention is an unsubstituted quinacridone pigment in the beta or gamma crystal phase as described in formula B. 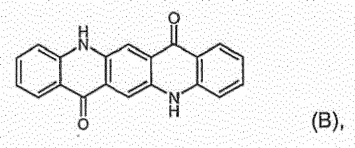 or salt thereof

### Brief description of the drawings

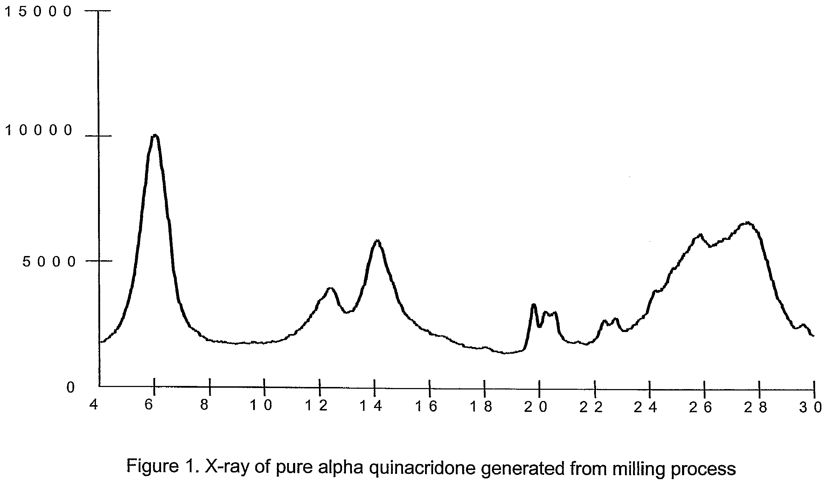

- Figure 1.: X-ray of pure alpha quinacridone.

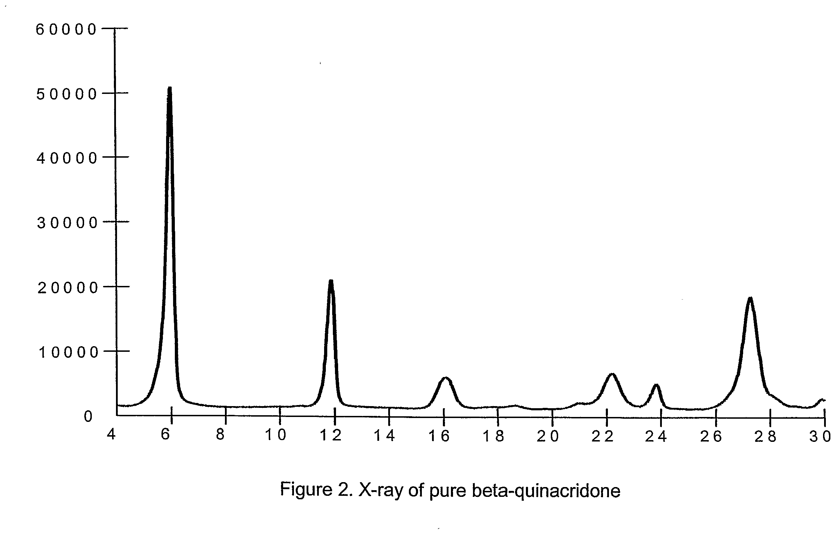

- Figure 2.: X-ray of pure beta-quinacridone.
- Figure 3.: X-ray of example B-1, aqueous wet-milled crude beta-quinacridone without 2,9-dichloroquinacridone.
- Figure 4.: X-ray of example B-2, aqueous wet-milled crude beta-quinacridone with 2,9-dichloroquinacrione.
- Figure 5.: X-ray of example B-3, aqueous wet-milled crude beta-quinacridone with 2,9-dichloroquinacridone.
- Figure 6.: X-ray of example B-4, aqueous wet-milled crude beta-quinacridone with 2,9-dichloroquinacridone.
- Figure 7.: X-ray of example B-5, aqueous wet-milled crude beta-quinacridone with 2,9-dichloroquinacridone.
- Figure 8.: X-ray of comparison example B-1.
- Figure 9.: X-ray of comparison example B-2.
- Figure 10.: X-ray of comparison example B-3.

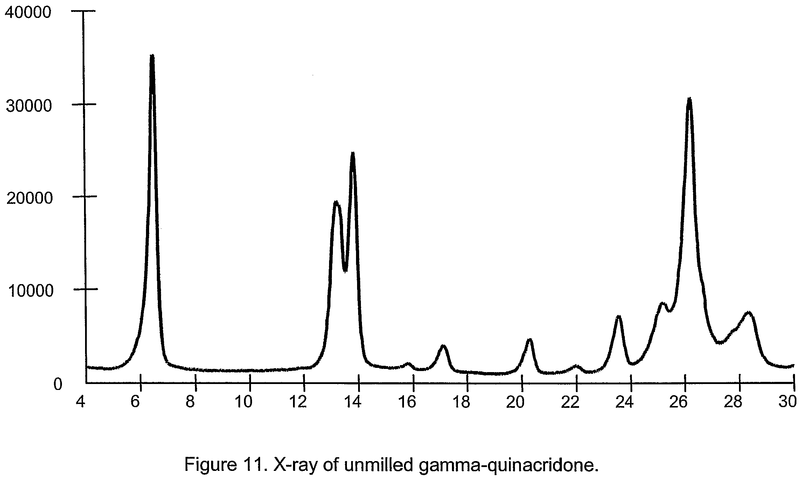

- Figure 11.: X-ray of unmilled gamma-quinacridone.

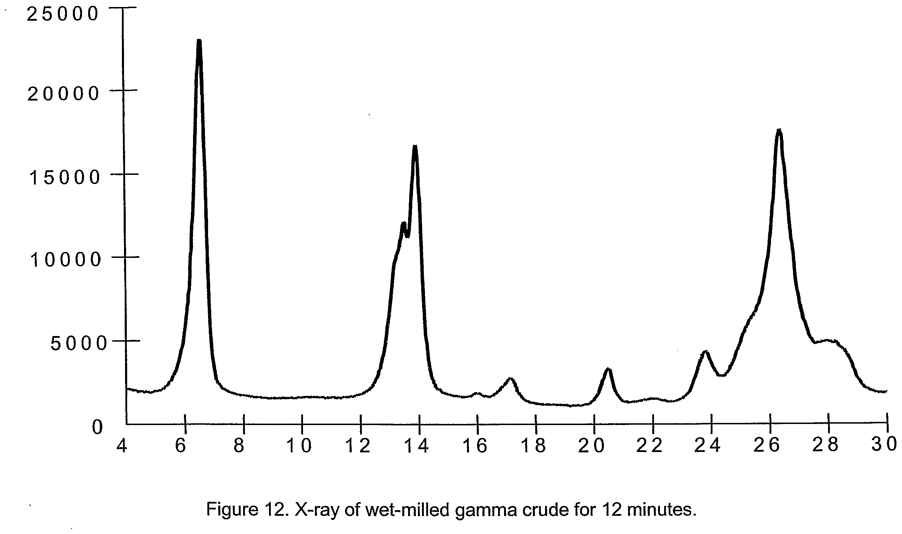

- Figure 12.: X-ray of aqueous wet-milled gamma crude for 12 minutes.

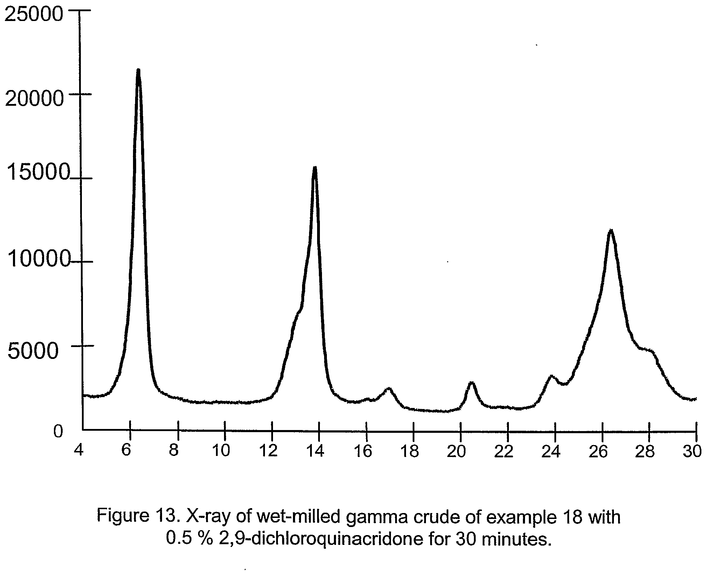

- Figure 13.: X-ray of aqueous wet-milled gamma crude of example 18 with 0.5 % 2,9-dichloroquinacridone for 30 minutes.

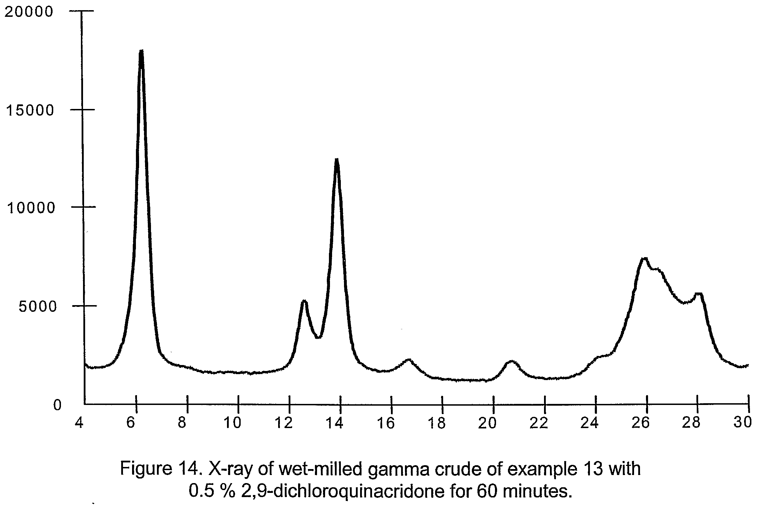

- Figure 14.: X-ray of aqueous wet-milled gamma crude of example 13 with 0.5 % 2,9-dichloroquinacridone for 60 minutes.

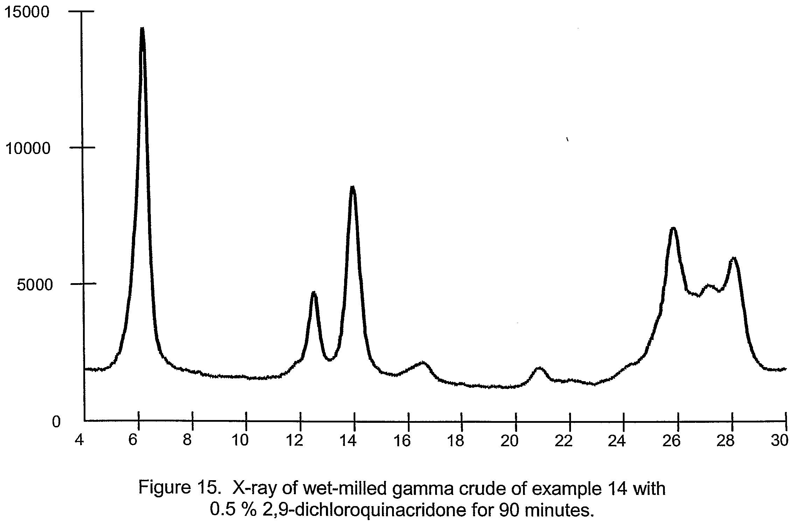

- Figure 15.: X-ray of aqueous wet-milled gamma crude of example 14 with 0.5 % 2,9-dichloroquinacridone for 90 minutes.
- Figure 16.: X-ray of aqueous wet-milled gamma crude of example 16 with 0.5 % 2,9-dichloroquinacridone for 120 minutes.

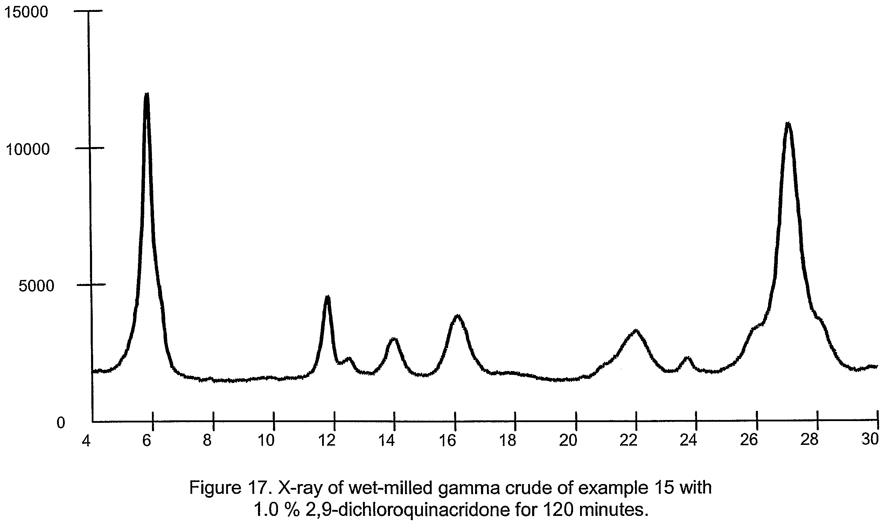

- Figure 17.: X-ray of aqueous wet-milled gamma crude of example 15 with 1.0 % 2,9-dichloroquinacridone for 120 minutes.

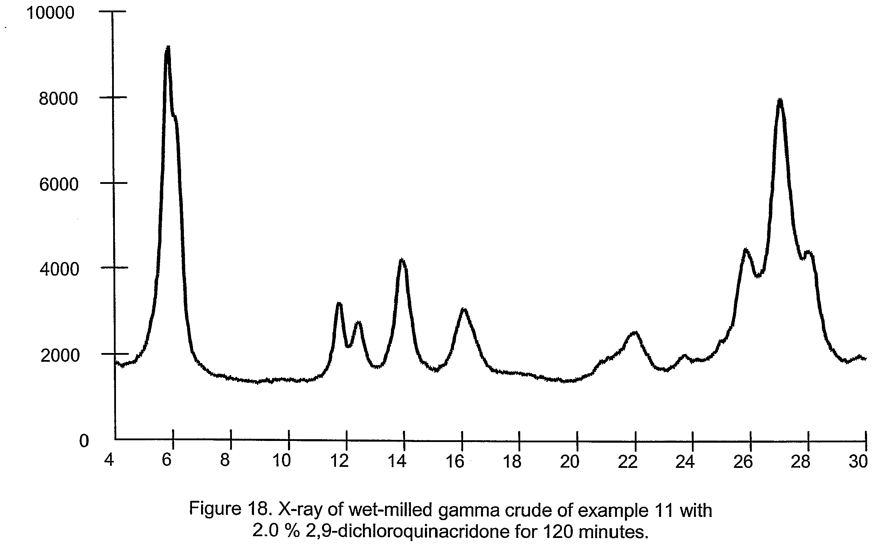

- Figure 18.: X-ray of aqueous wet-milled gamma crude of example 11 with 2.0 % 2,9-dichloroquinacridone for 120 minutes.

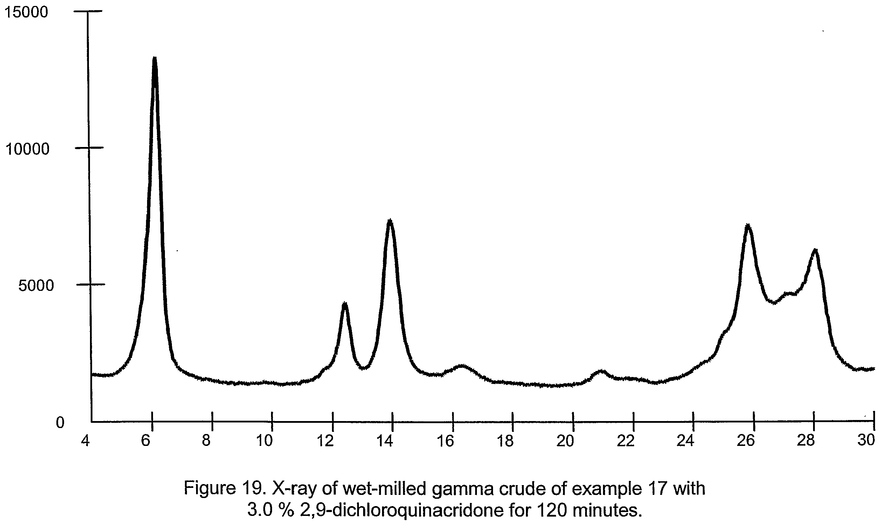

- Figure 19.: X-ray of aqueous wet-milled gamma crude of example 17 with 3.0 % 2,9-dichloroquinacridone for 120 minutes.
- Figure 20.: X-ray of aqueous wet-milled beta crude of example 6 with 0.5 % 2,9-dichloroquinacridone for 60 minutes.
- Figure 21.: X-ray of aqueous wet-milled beta crude of example 7 with 0.5 % 2,9-dichloroquinacridone for 120 minutes.
- Figure 22.: X-ray of aqueous wet-milled beta crude of example 8 with 1.0 % 2,9-dichloroquinacridone for 60 minutes.
- Figure 23.: X-ray of aqueous wet-milled beta crude of example 9 with 1.0 % 2,9-dichloroquinacridone for 120 minutes.
- Figure 24.: X-ray of aqueous wet-milled beta crude of example 10 with 2.0 % 2,9-dichloroquinacridone for 60 minutes.

The crude beta- or gamma-pigments are generally those lacking in properties required for a colorant because of inferior color development and having a particle diameter size range of about .2 to 40 µm, preferably .3 to 4 µm, most preferably about 1.0-3.0 µm. Alternatively, in the instant invention, commercially available pigments composed of pigment particles with a particle diameter size range of about 0.3 to 0.5 µm may be used as the raw materials to be milled.

In particular, the unsubstituted quinacridones of formula (B) in crude beta and gamma crystal phases are especially preferred.

Aqueous milling may be carried out using known wet-milling methods. Although the particular milling apparatus is generally not critical, suitable mills include horizontal mills, for example, Dyno-mill, Eiger mills, Netzsch mills, and Super mills. Additional vertical mills, ball mills, attritors, vibratory mills, and the like containing various grinding media are suitable. Suitable grinding media include salt, sand, glass beads, ceramic beads and alumina, zirconium or metal beads.

Milling may also be carried out by dispersion milling. A commercial dispersion milling process for quinacridone pigments wherein the particle size thereof is reduced is disclosed in U.S. Pat. No. 3,030,370. The process involves milling in the presence of anhydrous aluminum sulfate and in the presence of a crystallizing solvent. The crystallizing solvents are broadly defined as anhydrous organic solvents with boiling ranges high enough to withstand the heat of grinding without volatilization and low enough to permit removal by steam distillation. Suitable solvents include tetrachloroethylene, other hydrocarbons and chlorinated hydrocarbons and lower alkyl esters of C₂-C₁₀ dibasic carboxylic acids can also be readily utilized as crystallizing solvents in such dispersion milling processes.

Grinding in the absence of solvents tends to convert the products to the least stable phases (alpha phase). The introduction of a solvent alters the equilibrium but the degree of alteration is influenced by the nature and amount of solvent, the nature of the pigment, and the amount of grinding. The solvent tends to promote the formation of the more stable phases or, as a corollary, to retain the more stable phase if it is the starting material.

Regardless of the type of milling used (wet-milling or dispersion milling), the process of the invention has surprisingly discovered that milling of crude beta- or gamma-quinacridone pigment with 2,9-dichloroquinacridone either maintains the beta or gamma phase and/or prevents the starting beta or gamma phase from converting to the less stable alpha phase.

Milling temperature depends on the size of the mill, and the quantity of crude pigment being milled but is generally carried out at a temperature of 20°C to about 95°C. Preferably the process milling temperature Is 30 °C to about 90 °C. Optionally, cooling with water may control the temperature.

In this embodiment of the instant invention, the average particle diameter of the resulting milled pigments are about 30-300 nm, preferably 40-200 nm.

Particle size reduction time for either the beta-quinacridone or gamma-quinacridone in the presence of the crystal phase inhibitor, 2,9-dicholorquinacridone may vary from thirty minutes to forty-eight hours depending upon the particle size needed for a particular application and the particular crude pigment being wet-milled or dispersion milled.

Grinding media for wet-milling is generally loaded to about 75%-85% of chamber space. The milling media, consists of beads composed of materials such as zirconium oxide, glass, borosilicate, metal, alumina and polymeric beads for example, those described in U.S. Patent Nos. 5,902,711, and 5,478,705.

Grinding media for dispersion milling is generally steel shot, iron nails and spikes, or ceramic beads. Dispersion milling cycles generally range from about 2 to about 48 hours. The amount of solvent is chosen such that the desired crystal phase is maintained, while allowing the desired particle size to be generated in a reasonable mill time. Amounts ranging from 2 to 15%, by weight of quinacridone, and preferably 4 to 13%, are generally utilized.

Suitable milling liquid for wet-milling is water, and can include less than 5 wt. % of polar organic solvent, such as dimethylformamide, dimethylacetamide, dimethylsulfoxide, lower aliphatic alcohols such as methanol, ethers including tetrahydrofuran and dioxane, and alkylene glycols and triols such as ethylene glycol and glycerol.

The wet-milling liquid is made up of greater than about 10 wt. % water, preferably greater than about 20 wt.% water, and most preferably greater than 30 wt. % water.

The milling may take place at a pH that ranges from about 4.0 to about 12.0. The pH is preferably about 6 to about 9.

The dispersion-milling may be carried out in an appropriate crystallizing solvent. The crystallizing solvents are broadly defined as anhydrous organic solvents with boiling ranges high enough to withstand the heat of grinding without volatilization and low enough to permit removal by steam distillation. Suitable solvents include tetrachloroethylene, other hydrocarbons and chlorinated hydrocarbons and lower alkyl esters of C₂ -C₁₀ dibasic carboxylic acids can also be readily utilized as crystallizing solvents in such dispersion milling processes.

Colored additives, such as organic pigment derivatives, or uncoloured additives, such as polymers, can also optionally be added to the milling mixture during the milling process.

The crude pigment may be optionally wet-milled or dispersion milled with other additives such as surface modification reagents, rheology improving agents, texture improving agents, defoamers, wetting agents, particle growth inhibitors, other crystal phase directors, antiflocculants, polymeric wet-milling aids and dispersants.

Surface modifying reagents, rheology improving agents and texture improving agents may include quinacridone monosulfonic acid or quinacridone monosulfonic acid aluminum salt, 3,5-dimethylpyrazol-1-methyl quinacridone or phthalimidomethyl quinacridone. Other suitable texture improving agents are, in particular, fatty acids of not less than 18 carbon atoms, for example stearic or behenic acid or their amides or metal salts thereof, preferably sodium or ammonium salts, as well as plasticizers, waxes, resin acids such as abietic acid or metal salts thereof, colophonium, alkyl phenols or aliphatic alcohols such as stearyl alcohol or vicinal diols such as dodecane-1, 2-diol. The additives may be added directly to the milling slurry or at the same time as the crude pigment. The additive or additives may optionally be added at about 0.5-20.0 wt. % based on the dry weight of the crude organic pigment. Preferably the additive or additives are optionally added at about 1,0-5.0 wt. % based on the dry weight of the crude organic pigment.

Defoamers can be used optionally in the wet-milling process of this embodiment of the instant invention. The defoamer may be added before and/or during milling for foam control.

Dispersing agents or polymeric grinding aids for wet-milling may be styrenic resins such as those described in copending U.S. Application No. 60/519,842 or acrylic resins such as those described in U.S. Patent No.6,410,619.

After milling, the pigment may be separated from the milling mixture by one or more isolation methods known in the art. Filtration, followed by washing to remove residual salts and solvent, is the preferred separation method. Other collection methods known in the art, such as tray drying, spray drying, spin flash drying, lyophilization, centrifugation, or simple decantation are also suitable isolation methods. Such methods can be used individually or in combination.

The present reduced size gamma and/or beta quinacridone pigments are suitable as coloring matter for inorganic or organic substrates. They are highly suitable for coloring high molecular weight materials, which can be processed to casted and molded articles or which are used in ink and coating compositions such as solvent or water based coatings, for example in automotive coatings. Preferred high molecular weight materials are plastics that are subsequently calendered, cast, molded or processed to fibers and industrial or automotive paints or ink coatings.

For the purposes of the invention, high molecular weight material is defined as material in the range of 10³ to 10⁸ g/mol.

Suitable high molecular weight organic materials include thermoplastics, thermoset plastics or elastomers, for example, cellulose ethers; cellulose esters such as ethyl cellulose; linear or crosslinked polyurethanes; linear, crosslinked or unsaturated polyesters; polycarbonates; polyolefins such as polyethylene, polypropylene, polybutylene or poly-4-methylpent-1-ene; polystyrene; polysulfones; polyamides; polycycloamides; polyimides; polyethers; polyether ketones such as polyphenylene oxides; and also poly-p-xylene; polyvinyl halides such as polyvinyl chloride, polyvinylidene chloride, polyvinylidene fluoride or polytetrafluoroethylene; acrylic and methacrylic polymers such as polyacrylates, polymethacrylates or polyacrylonitrile; rubber; silicone polymers; phenol/formaldehyde resins; melamine/formaldehyde resins; urea/formaldehyde resins; epoxy resins; diene rubbers or copolymers thereof such as styrene butadiene rubber; acrylonitrile-butadiene rubber or chloroprene rubber; singly or in mixtures.

Generally, the present reduced size gamma and/or beta quinacridone pigments are used in an effective pigmenting amount, for example, of 0.01 to 30% by weight, preferably 0.1 to 10% by weight, based on the weight of the high molecular weight organic material to be pigmented. Thus, the present invention also relates to a pigmented plastic composition, which comprises a plastic material and an effective pigmenting amount of a pigment or pigment solid solution prepared according to a process of the present invention, and to a process for preparing said pigmented plastic compositions.

The present reduced size gamma and/or beta quinacridone pigments are easily dispersible and can be readily incorporated into organic matrixes to provide homogenous colorations possessing high saturation.

The high molecular weight organic materials are pigmented with the present reduced size gamma and/or beta quinacridone pigments by mixing the pigments, if desired in the form of a masterbatch, into substrates using high shear techniques including roll mills or a mixing or grinding apparatus. The pigmented material is then brought into the desired final form by known methods, such as calandering, pressing, extruding, brushing, casting or injection molding.

In yet another embodiment, the instant invention relates to a process for particle size reduction of beta- or gamma-quinacridone pigment crystals while maintaining the beta or gamma crystal phase to improve the optical properties of said pigments wherein the process comprises
(a) milling a mixture comprising:
   i.) about 10-60 wt. % of a composition comprising 2,9-dichloroquinacridone with beta or gamma-quinacridone wherein the wt. % of the 2,9-dichloroquinacridone is about 0.1 % to about 5.0 weight % based on the dry weight of the beta or gamma pigment,
   ii.) about 0.1-25 wt. % of a water-soluble styrene copolymer dispersant based on the dry weight of the composition comprising 2,9-dichloroquinacridone with beta or gamma-quinacridone,
   iii.) about 0.1-1.0 wt. % of defoamer,
   iv.) optionally about 0.1-5.0 wt. % of an additive based on the dry weight of the composition comprising 2,9-dichloroquinacridone with beta or gamma-quinacridone, and
   v.) greater than about 10 wt. % of water; wherein the weight percent of components i.), iii.) and v.) are based on the total weight of the mixture, wherein the mixture is continuously circulated through a milling media,
(b) isolating the beta- or gamma-quinacridone pigment crystals.

Preferably, the mixture comprises
i.) about 10-45 wt. % of a composition comprising 2,9-dichloroquinacridone with beta or gamma-quinacridone wherein the wt. % of the 2,9-dichloroquinacridone is about 0.1 % to about 5.0 weight % based on the dry weight of the beta or gamma pigment,
ii.) about 1.0-20 wt. % of a water-soluble styrene copolymer dispersant based on the dry weight of the composition comprising 2,9-dichloroquinacridone with beta or gamma-quinacridone,
iii.) about 0.1-1.0 wt. % of defoamer,
iv.) optionally about 0.1-5.0 wt. % of an additive based on the dry weight of the composition comprising 2,9-dichloroquinacridone with beta or gamma-quinacridone, and
v.) greater than about 10 wt. % of water; wherein the weight percent of components i.), iii.) and v.) are based on the total weight of the mixture.

More preferably, the mixture comprises
i.) about 15-35 wt. % a composition comprising 2,9-dichloroquinacridone with beta or gamma-quinacridone wherein the wt. % of the 2,9-dichloroquinacridone is about 0.1 % to about 5.0 weight % based on the dry weight of the beta or gamma pigment,
ii.) about 5-10 wt. % of a water-soluble styrene copolymer dispersant based on the dry weight of the composition comprising 2,9-dichloroquinacridone with beta or gamma-quinacridone,
iii.) about 0.1-1.0 wt. % of defoamer,
iv.) optionally about 0.5-5.0 wt. % of an additive based on the dry weight of the composition comprising 2,9-dichloroquinacridone with beta or gamma-quinacridone, and
v.) greater than about 30 wt. % of water; wherein the weight percent of components i.), iii.) and v.) are based on the total weight of the mixture.

### Examples A

The aqueous particle size reduction process is performed using circulation milling media, such as zirconium oxide beads in a Dyno-mill and Netzsch-mill, in a size range of about 0.2-1.2 mm diameter with a loading of 75-85% of milling chamber space. Other than water, the milling slurry has a composition of about 10-60 wt. % organic pigment or mixture of pigments based on the total weight of the composition, about 0.1-25 wt. % of styrene copolymer dispersant based on the dry weight of the pigment and 0.1-1 wt. % defoamer based on the total weight of the composition. Optionally, the milling media may have about 0.1-5 wt. % non-pigment milling additive, pigment additive or mixtures of additives based on the dry weight of the pigment. Particle size reduction time may vary from about thirty minutes to about twelve hours in order to achieve the particle size needed for the application. Milled product may be isolated after particle size reduction process, or the crystal size can be re-grown by heating the aqueous slurry to 70-95 °C with addition of base, polar organic solvent and / or amine salt.

Color data is obtained using CM-3600d spectrophotometer manufactured by Minolta Corporation USA. 101 Williams Drive, Ramsey, NJ.

In Example A-2 and Example A-5, heat stability data is obtained by: heating colored plastic chips for 5 minutes at 20 °C temperature intervals from 200 - 300 °C, then measuring the color change in the plastic chips using a CM-3600d spectrophotometer. As the temperature increases, a lower color difference (measured as dE in both masstone and tint) indicates better heat stability of the pigment used in coloring the plastics.

### Definitions

¹® Joncryl HPD-96 Styrene-acrylic copolymer. Molecular weight range of 8,500 - 16,500, acid number 215 - 240. Manufactured by SC Johnson Polymers, 8310 16th Street, P.O. Box 902, Sturtevant, WI.
²® Surfynol DF- 37 Acetylenic defoamer. Manufactured by Air Products, 7201 Hamilton Boulevard, Allentown, PA.
³® Sunfast 228-6725 - 2,9-dichloroquinacridone made by Sun Chemicals.
⁴Ciba ® Cinquasia Magenta RT-265-D- Ciba commercial product, 2,9-dichloroquinacridone pigment.
⁵Quindo Red R-6700 - Commercial product, manufactured by Bayer Corporation.
⁶Ciba ® Cinquasia Red RT-790-D- Ciba commercial product, gamma-quinacridone pigment.
⁷Ciba ® Cinquasia Red NRT-742-D - Ciba commercial product, gamma-quinacridone pigment.
⁸Ciba ® Cinquasia Red B NRT -796-D - Ciba commercial product, gamma-quinacridone pigment.
⁹Ciba ® Cinquasia Violet R NRT-201-D - Ciba commercial product, beta-quinacridone pigment.
¹⁰Ciba ® Cinquasia Violet R RT-791-D - Ciba commercial product, beta-quinacridone pigment.
¹¹Ciba ® Cinquasia Red Y RT -759-D - Ciba commercial product, gamma-quinacridone pigment.
¹²ZrO₂/SiO₂ beads are manufactured by Glen Mills Inc., 395 Allwood Road, Clifton, New Jersey, 07012.

### Example A-1 Crude 2,9-dichloroquinacridone aqueous milling

To a 4000ml plastic beaker is added 858.4g of 2,9-dichloroquinacridone press cake (46.6 wt. % solid), 391.6g of water, 37.5g of Joncryl HPD-96¹ (34 wt. % solid), and 3.9g of Surfynol DF-37² Defoamer. The above ingredients are mixed with a high shear stirrer (eg. a Ross Mixer) at a suitable setting for 5 minutes. The pH is adjusted to a range of 8.5-9.0 with 10% ammonium hydroxide. The resulting slurry is milled in the Dyno-mill.

The Dyno-Mill is filled with 1220g (500 ml) of 1.0-1.25 mm ZrO₂/SiO₂ beads (mill gap setting at 0.10, tip speed 30m/minute). The prepared pigment slurry is transferred to the Dyno-Mill until color comes out of the discharge tube. Milling is started at a flow rate of 80 ∼ 100 ml/minute. Addition of Joncryl HPD-96 and DF-37 are made as the milling continues in order to maintain fluid viscosity with a total of 45.5g Joncryl HPD-96 and 5.1g of Surfynol DF-37 used. Sampling is made each hour in order to monitor the milling process. Total milling time is 4.8hr, 42 passes are completed, and 1200ml slurry product of 30.2 wt. % solid is obtained that is used for the ripening experiment in Example A-2. Milled product shows higher chroma in both tint and masstone, and tint strength increases by 87% in comparison to the crude pre-milled pigment.

### Example A-2 Particle size re-grow process suitable for production of high performance opaque pigment after aqueous-milling

To a 1000ml flask is added 120ml of water, 24.0g of NaOH (50% solution), 3.6g 50% solution of benzyl tributylammonium chloride in hexylene glycol/water, and 7.2g of pentanol, and stirred at room temperature for 10 minutes. 60g slurry sample of Example A-1 of the milled quinacridone is added to above mixture with stirring at room temperature for 30 minutes, then heating to reflux (97 °C) for 4 hr.

The reaction mixture is cooled to 60 °C, transferred to a filtration funnel, filtered and washed with 0.1 % aqueous sulfuric acid, then washed with hot water to a pH of 7.0. 22.9g of 75.2 wt. % solid presscake sample are obtained. This sample is dried at 80 °C in an oven overnight, blended to the powder form, and submitted for paint and plastics screening.

Example A-2 is tested in base coat/clear coat paint using Sunfast Magenta 228-6725³ commercial product as standard. Example A-2 shows similar opacity, equal chroma in both masstone and tint, and bluer hue in masstone, with dE = 0.5 in tint.

Plastic PVC test shows similar trend with dE = 0.7 in tint. This data shows that Example A-2 has similar color shade and performance to Sunfast Magenta 228-6725³ in both paint and plastic applications. A heat stability test is performed using engineering plastics ABS media and Ciba commercial product ®CINQUASIA MAGENTA RT-265-D⁴ as standard. The pigmented ABS is tested for heat stability in temperature ranges from 200 °C to 300 °C. Example A-2 is more heat stable than RT-265-D in both tint and masstone, with masstone dE = 2 compared with masstone dE = 2.87 for RT 265-D in the 200 - 300 °C temperature range.

### Example A- 3 Crude gamma-quinacridone and 2,9-dichloroquinacridone mixture aqueous-mililng

To a 4000ml plastic beaker is added 972.7g of gamma-quinacridone presscake (40.3 wt. % solid), 17.2g of 2,9-dichloroquinacridone presscake (46.6 wt. % solid), 260.1g of water, 47.0g of Joncryl HPD-96 (34 wt. % solid), and 9.0g of Surfynol DF-37 defoamer. The above ingredients are mixed with a high shear stirrer (eg. a Ross Mixer) at a suitable setting for 15 minutes. The pH is adjusted to a range of 8.5-9.0 with 10% ammonium hydroxide. The resulting slurry is milled in the Dyno-Mill,

The Dyno-Mill is filled with 1220g (500ml) of 0.4-0.6 mm ZrO₂/SiO₂ beads (mill gap setting at 0.10, tip speed 30m/minute). The pigment slurry is transferred to the Dyno-Mill until color comes out of the discharge tube. The milling is started at a flow rate of 80 - 100 ml/minute. Total milling time is 2.5hr, 20 milling cycles are completed, and 1300ml pigment slurry of 31.5 wt. % solids is obtained. The slurry sample is used for pre-laboratory test using Latex paint card drawdown method.

### Example A-4 Isolation of example A-3 after aqueous-milling

To 500ml of Example A-3 is added 2500ml of water and pH is adjusted to 5.0 - 6.0 with 2% H₂SO₄. Sample is filtered and washed to pH = 7.0. Continued vacuum filtration increases the solids to 47 wt. %. Example A-4 is tested in a Latex paint system using Bayer R-6700⁵ as standard. Example A-4 shows same color space as Quindo Red R-6700⁵ with masstone dE = 0.3 and tint dE = 0.9.

### Example A-5 Sample preparation for Example A-3 to be tested in paint and plastics

To a 2000ml beaker is added 100g of slurry in Example A-3 (28.8 wt. % solid) and 1500ml of water. The mixture is pH adjusted to 5.0 with 2% H₂SO₄. The slurry is filtered and washed with water. The press cake is dried at 80 °C overnight obtaining 24g of sample. The sample is then Retsch milled to fine powder for testing in paint and plastic applications.

In the PVC test, Example A-5 shows 11 % higher tint strength than Ciba commercial product ®CINQUASlA RED B RT-790-D⁶ with similar color shade and a dE < 2.0 in both masstone and tint color. In engineering plastic HDPE (high density polyethylene), Example A-5 also shows similar color to RT-790-D, with much better heat stability. While temperature increases from 200 °C to 300 °C, dE of Example A-5 in both masstone and tint is less than 1.5. compared to RT-790-D with dE = 9.92 in masstone, and dE = 12.1 in tint.

In base coat clear coat paint, Example 5 is compared with Ciba commercial products ®CINQUASlA RED B NRT-742-D⁷ and ®CINQUASIA RED B NRT-796-D⁸. Results show Example A-5 is more transparent than both standards, similar in color shade with tint / masstone dE < 1.5, and metallic dE = 2.0 compared to RT-796-D. In latex decorative paint application, Example A-5 has higher strength than RT-796-D in both deep and light tones, similar color shade and slightly higher in chroma.

### Example A-6 Crude beta-quinacridone aqueous milling

To a 4000ml plastic beaker is added 770.7g of beta-quinacridone presscake (51.9 wt. % solid), 479.3g of water, 37.5g of Joncryl HPD-96 (34 wt. % solid), and 3.9g of Surfynol DF-37 defoamer. The above ingredients are Ross mixed at a setting of 5 for 15 minutes. The pH is adjusted to a range of 8.5-9.0 with 10% ammonium hydroxide to prepare slurry for milling in the Dyno-mill.
The Dyno-Mill is filled with 1220g (500ml) of 0.4 - 0.6mm ZrO₂/SiO₂ beads (mill gap setting at 0.10, tip speed 30m/minute). The pigment slurry is transferred to the Dyno-Mill until color comes out of the discharge tube with a starting mill flow rate of 80 ∼ 100 ml/minute. Addition of Joncryl HPD-96 and DF-37 are made as the milling continues in order to maintain the fluidity with a total of 111.5g Joncryl HPD-96 and 6.9g of DF-37 used. Sampling is made each hour in order to monitor the milling process. Total milling time is 12.3hr, 96 milling cycles are completed, and 1200ml slurry product of 32.0 wt. % solid is obtained. The slurry product is pH adjusted to ∼5.0, filtered and washed with hot water to pH ∼7.0. The press cake sample is dried at 80 °C in an oven overnight, then Retsch milled to a fine powder for paint and plastic screening.

The product of Example A-6 is compared with Ciba commercial product ®CINQUASlA VIOLET R NRT-201-D⁹ in base coat / clear coat paint, and shows a similar color shade to NRT-201-D with dE = 0.4 in tint. It is more transparent than NRT-201-D in masstone, and slightly yellower in metallic color.

Example A-6 is also compared with Ciba commercial product ®CINQUASIA VIOLET R RT-791-D¹⁰ in PVC plastics. Compared with RT-791-D, Example A-6 is identical in tint color with higher tint strength, and higher chroma than RT-791-D in masstone with dE = 3.5.

### Example A-7

Example A-6 is repeated except that 0.3-0.4 mm ZrO₂/SiO₂ beads are used in the milling process, and experiment is stopped at 8 hour. A product similar to Example A-6 is obtained.

### Example A-8 Crude gamma-quinacridone aqueous milling

To a 4000ml plastic beaker is added 992.6g of gamma-quinacridone presscake (40.3 wt. % solid), 386.1g of water, 48.8g of Joncryl HPD-96 (34 wt. % solid), and 3.9g of Surfynol DF-37 Defoamer. The above ingredients are Ross mixed at a setting of 5 for 15 minutes. The pH is adjusted to 9.4 with 10% ammonium hydroxide and the prepared slurry is milled in the Dyno-mill.

The Dyno-Mill is filled with 1220g (500ml) of 0.4 - 0.6mm ZrO₂/SiO₂ beads (mill gap setting at 0.10, tip speed 30m/minute). The prepared pigment slurry is transferred to the Dyno-Mill until color comes out of the discharge tube. Milling is started at a flow rate of 80 - 100 ml/minute. Addition of Joncryl HPD-96 and DF-37 are made as the milling continues in order to maintain fluidity, total of 64.8g Joncryl HPD-96 and 7.8g of DF-37 is used. Sampling is made each hour in order to monitor milling process. Total milling time is 5hr. 1300ml product with 25.6 wt. % solid content is obtained. Although starting gamma-quinacridone has gamma-II phase, as milling continues, the crystals change phase to gamma-I. The final product has 100 wt. % gamma-I crystal phase with a bluer tint color, more transparent, and higher chroma in both tint and masstone color than the starting pigment Ciba ® Cinquasia Red Y RT-759-D¹¹.

### Example A-9 Crude perylene aqueous milling

To a 4000ml plastic beaker is added 1295.3 of perylene (Lot # MO2326D) presscake (38.6 wt. % solid), 434.2 g of water, 73.5 g of Joncryl HPD-96 (34 wt. % solid), and 5.0 g of Surfynol DF-37 Defoamer. The above ingredients are mixed with a high shear stirrer for 15 minutes. The pH is adjusted to 9.4 with 10% ammonium hydroxide and the prepared slurry is milled in the Dyno-mill.

The Dyno-Mill is filled with 1170g of 0.4 - 0.6mm ZrO₂/SiO₂ beads (Mill Gap setting at 0.10, tip speed 30M/minute). The prepared pigment slurry is transferred to Dyno-Mill until color comes out of the discharge tube. Milling is started at a flow rate of 80 ∼ 100 ml/minute. Addition of Joncryl HPD-96 and DF-37 are made as the milling continues in order to maintain fluidity, a total of 238.2 g Joncryl HPD-96 (34 wt. % active ingredients) and 7.0g of DF-37 is used. Sampling is made each hour in order to monitor milling process. Total milling time is 5hr. 1300ml pigment slurry with a 25.7 wt. % solid content is obtained.

**Comparison of wet-milled crudes with the starting crude in a latex paint system**

| Sample | % Strength | *DC*. |
|---|---|---|
| | Tint | Masstone |
| 2,9-dichloroquinacridone crude (Control) | 100.00 | 0.00 |
| Example A-1 | 187.10 | 0.14 |
| | | |
| gamma-quinacridone | | |
| and 2,9-dichloroquinacridone crude mix (Control) | 100.00 | 0.00 |
| Example A-3 | 115.25 | 2.35 |
| beta-quinacridone crude (Control) | 100.00 | 0.00 |
| Example A-6 | 164.33 | -3.87 |
| | | |
| beta-quinacridone crude (Control) | 100.00 | 0.00 |
| Example A-7 | 166.93 | -1.22 |
| | | |
| gamma-quinacridone crude (Control) | 100.00 | 0.00 |
| Example A-8 | 127.64 | 2.26 |
| | | |
| Perylene crude (Control) | 100.00 | 0.00 |
| Example A-9 | 123.60 | 2.29 |

### Latex Paint Formulation

| | |
|---|---|
| Deep Base: | PREMIUM PLUS® Interior Semi-Gloss Enamel, #5340, 100% acrylic latex. |
| | BEHR Process Corporation, 3400 W. Segerstrom Ave., Santa Ana, CA 92704. |
| Pastel Base: | PREMIUM PLUS® Interior Semi-Gloss Enamel, #5560, 100% acrylic latex. |
| | BEHR Process Corporation, 3400 W. Segerstrom Ave., Santa Ana, CA 92704. |

Masstone sample is made by mixing 2.5g (30 wt. % pigment loading in water) milled slurry product with 47.5g of Deep Base carrier.

Tint sample is made by mixing 0.6g (30 wt. % pigment loading in water) of milled slurry sample with 50g of Pastel Base carrier.

*DC, chroma change.

### EXAMPLES B (the examples of category "B" do not belong to the invention)

### General wet-milling procedure

The aqueous particle size reduction process (wet-milling) for the crude beta-quinacridone is performed using circulation milling media, such as zirconium oxide beads in a Netzsch-mill and Dyno-mill, in a size range of about 0.2-1.2 mm diameter with a loading of about 75 to about 90% of milling chamber space. Other than water, the milling slurry has a composition of about 5 to about 45% organic pigment or mixture of pigments based on the total weight of the slurry. Optionally, the milling media may have about 0.5 to about 20% pigment derivative additives, based on the dry weight of the pigment, non-pigment additives including polymeric dispersants or mixtures of additives. Particle size reduction time may vary from about ten minutes to about forty-eight hours in order to achieve the particle size needed for the application. Milled product may be isolated after particle size reduction process, or the crystal size can be re-grown by heating the aqueous slurry to about 70 to about 95 °C with addition of base, polar organic solvent and/or amine salt.

### General dispersion milling procedure

The disperson milling process for the crude gamma or beta-quinacridone is preformed by charging a commercial ball mill with "Cyl-Pebs®" (approximately 2.5 cm sections of 1.6 cm diameter steel rod) and railroad spikes along with commercial aluminum sulfate and a crystallizing solvent. In the present example the crystallizing solvent is dimethyl glutarate. The crude gamma or beta-quinacridone is then charged into the ball mill. The charge is ground by rotating the mill for about 2 to about 48 hours. The contents of the mill are then discharged through a screen which retains the "Cyl-Pebs®" and railroad spikes.

### Extraction after dispersion milling of beta or gamma-quinacridone

A suitable vessel is charged with 1.5% sulfuric acid and the milled content above. The mixture is heated to about 90° C. The pigmentary solution of gamma-quinacridone is isolated in a suitable filtration device and washed free of acid and salts. The resulting water wet pigment may be either dried or further treated depending on the desired end use.

Color data is obtained using CM-3600d spectrophotometer manufactured by Minolta
Corporation USA. 101 Williams Drive, Ramsey, NJ.

### Beta-Quinacridone

### EXAMPLE B-1

### Crude beta-quinacridone aqueous milling with phthalimidomethyl quinacridone, without 2,9-dichloroquinacridone

To a 5000 ml circulation flask is added 200.0g of dry beta-quinacridone crude, 32.0g of polymeric dispersant Scripset 720 (25% maleic acid acrylic acid copolymer in water) manufactured by Hercules Incorporated, Wilmington, Delaware, 8.0g phthalimidomethyl quinacridone, and 500.0g of water. Above chemicals are mixed with a mechanic stirrer for 30 minutes. The resulting slurry is milled in a Netzsch Mill in a pH range of 7.0-9.0.

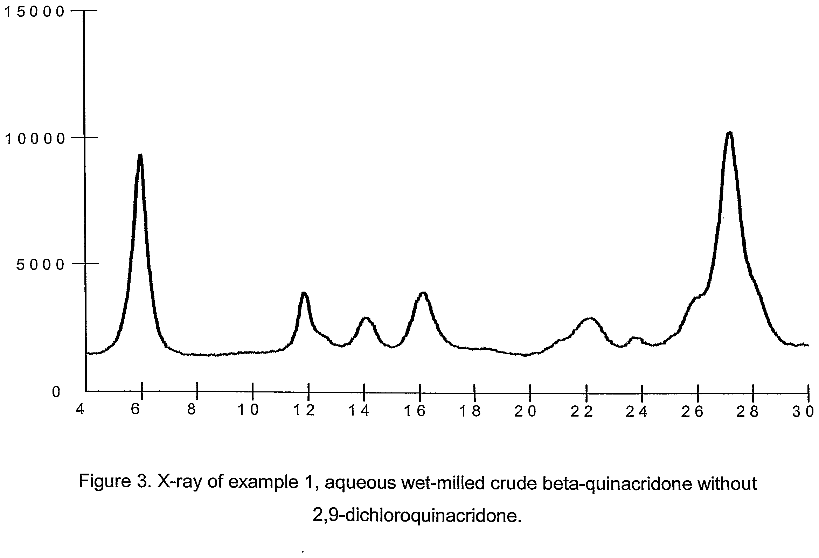

The Netzsch Mill grinding chamber is filled with 1825.0g (500 ml) of 0.3 mm ZrO₂/Y₂O₃ beads and 367.0g of water. Prepared pigment slurry is pumped into the Netzsch Mill, milling is started at flow rate of 400.0g/minute, tip speed set up at 12.0M/minute, and milling temperature is controlled in a range of 50 - 55 °C. Total milling time is 120 minutes. Milled beta-quinacridone slurry is diluted with 500.0g of water, slurry pH is adjusted to 5.0 with 2% H₂SO₄, then filtered and washed with hot water till pH 7.0, dried in an oven at 80 °C. The isolated product is submitted for screening in alkyd melamine paint. X-ray of the milled product shows a peak at 14θ corresponding to alpha-quinacridone. Color data of beta-quinacridone crude and the milled product is listed in Table 3. X-ray of the milled crude beta-quinacridone without 2,9-dichloroquinacridone product is shown Figure 3.

### EXAMPLE B-2

### Crude beta-quinacridone aqueous milling with phthalimidomethyl quinacridone and 2,9-dichloroquinacridone

To a 5000 ml circulation flaks is added 171.5g of dry beta-quinacridone crude, 3.5g of phthalimidomethyl quinacridone, 3.5g of 2,9-dichloroquinacridone, and 1951.5g of water. Above chemicals are mixed with a mechanic stirrer for 30 minutes. The resulting slurry is milled in a Netzsch Mill in a pH range of 7.0-9.0.

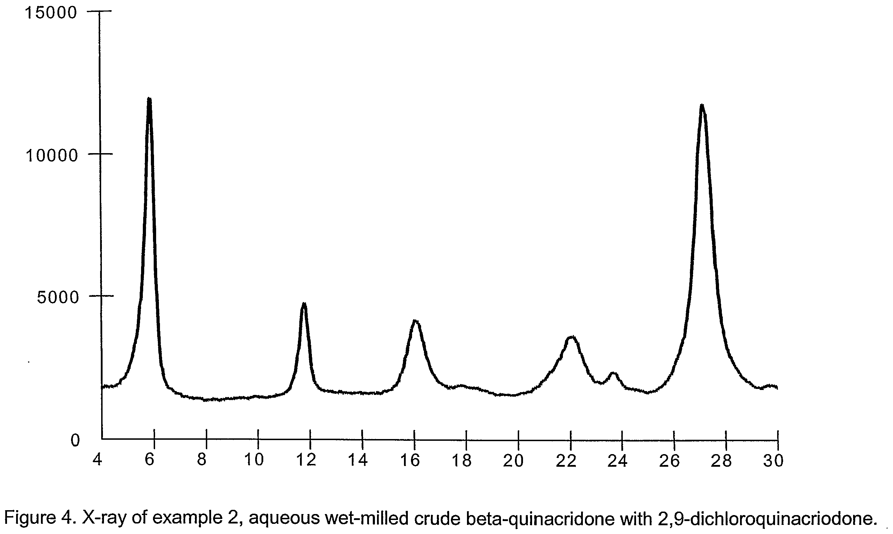

The Netzsch Mill grinding chamber is filled with 1825.0g (500 ml) of 0.3 mm ZrO₂/Y₂O₃ beads and 370.0g of water. Prepared pigment slurry is pumped into the Netzsch Mill, milling is started at flow rate of 800.0g/minute, tip speed set up at 12.0M/minute, and milling temperature is controlled in a range of 80 - 85 °C. Total milling time is 102 minutes. 500.0g of milled beta-quinacridone slurry is diluted with 500.0g of water, then filtered and washed with hot water till pH 7.0, dried in an oven at 80 °C. The isolated product is submitted for screening in alkyd melamine paint. X-ray of the milled product shows no peak at 14θ, which indicates no alpha-quinacridone. Color data of the milled product is listed in Table 3. X-ray of the milled product is shown in Figure 4.

### EXAMPLE B-3

### Crude beta-quinacridone aqueous milling with 2,9-dichloroquinacridone

To a 5000 ml circulation flaks is added 171.5g of dry beta-quinacridone crude, 3.5g of 2,9-dichloroquinacridone 14.0g of Staybelite Ester®, a polymeric dispersant 10-55WK (55% active aqueous dispersion of the glycerol ester of hydrogenated rosin mixture, manufactured by Eastman Chemical Resins, Kingsport, Tennessee), and 1955.0g of water. Above chemicals are mixed with a mechanic stirrer for 30 minutes. The resulting slurry is milled in a Netzsch Mill in a pH range of 7.0-9.0.

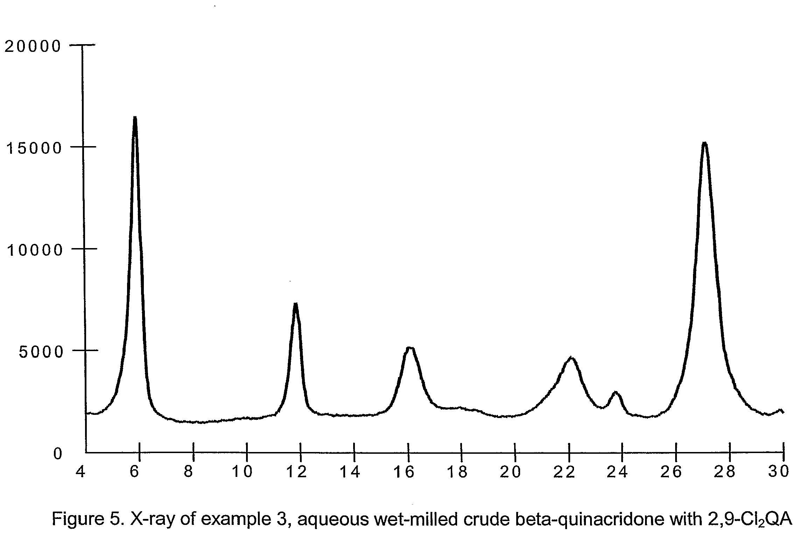

The Netzsch Mill grinding chamber is filled with 1825.0g (500 ml) of 0.3 mm ZrO₂/Y₂O₃ beads and 370.0g of water. Prepared pigment slurry is pumped into the Netzsch Mill. Milling is started at flow rate of 800.0g/minute, tip speed set up at 12.0M/minute, and milling temperature is controlled in a range of 80 - 85 °C. Total milling time is 60 minutes. 300.0g of milled beta-quinacridone slurry is diluted with 500.0g of water, then filtered and washed with hot water till pH 7.0, dried in 80 °C oven overnight. The isolated product is submitted for screening in alkyd melamine paint. X-ray of the milled product shows no peak at 14θ, which indicates no alpha-quinacridone. Color data of the milled product is listed in Table 3. X-ray of the milled product is shown in Figure 5.

### EXAMPLE B-4

### Crude beta-quinacridone aqueous milling with 2,9-dichloroquinacridone.

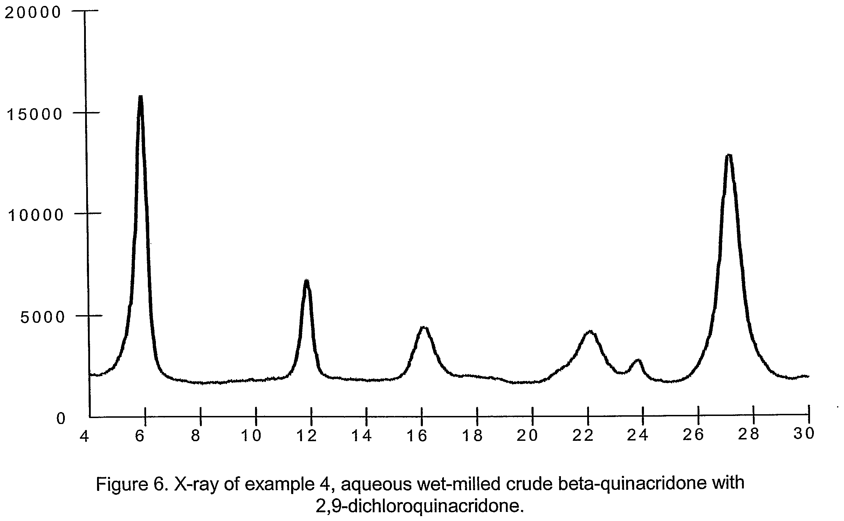

Example B-3 is repeated except; milling time is 90 minutes. The isolated product is submitted for screening in alkyl melamine paint. X-ray of the milled product shows no peak at 14θ, which indicates no alpha-quinacridone. Color data of the milled product is listed in Table 3. X-ray of the milled product is shown in Figure 6.

### EXAMPLE B-5

### Crude beta-quinacridone aqueous milling with 2,9-dichloroquinacridone.

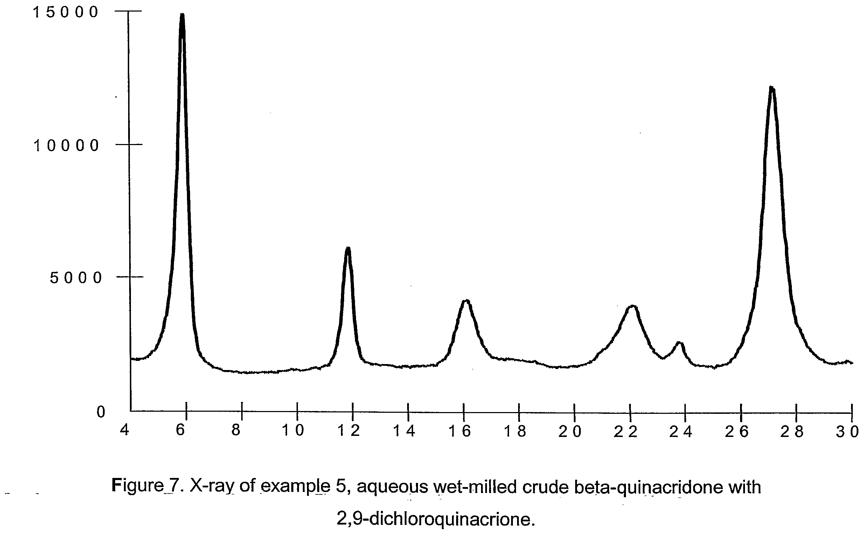

Example B-3 is repeated except; milling time is 120 minutes. The isolated product is submitted for screening in alkyd melamine paint. X-ray of the milled product shows no peak at 14θ, which indicates no alpha-quinacridone. Color data of the milled product is listed in Table 3. X-ray of the milled product is shown in Figure 7.

### EXAMPLES B-6 to B-11

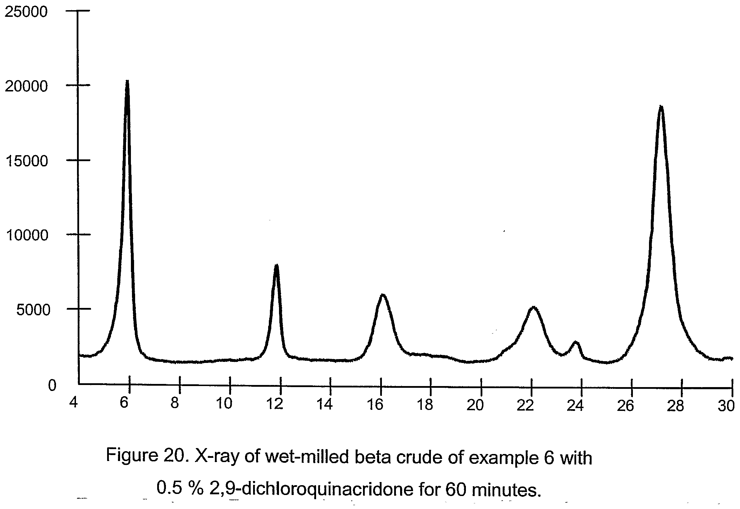

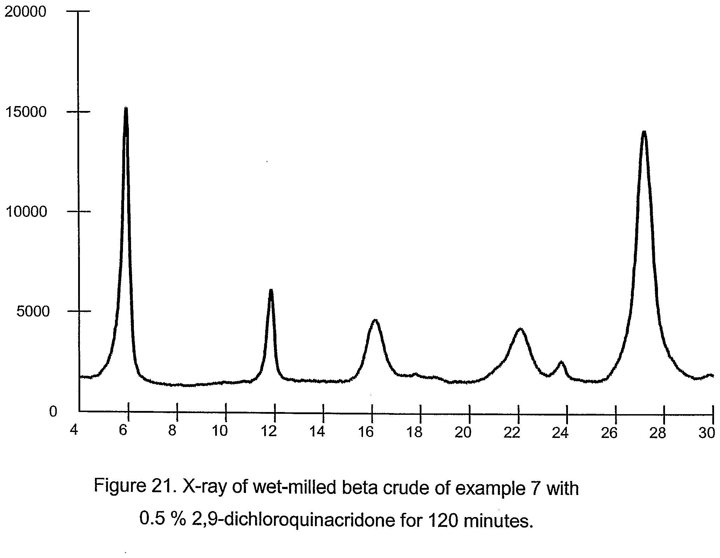

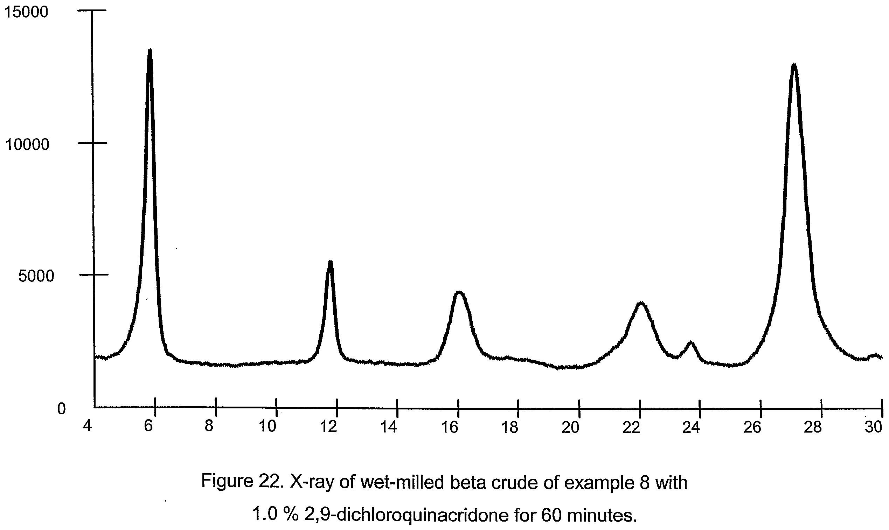

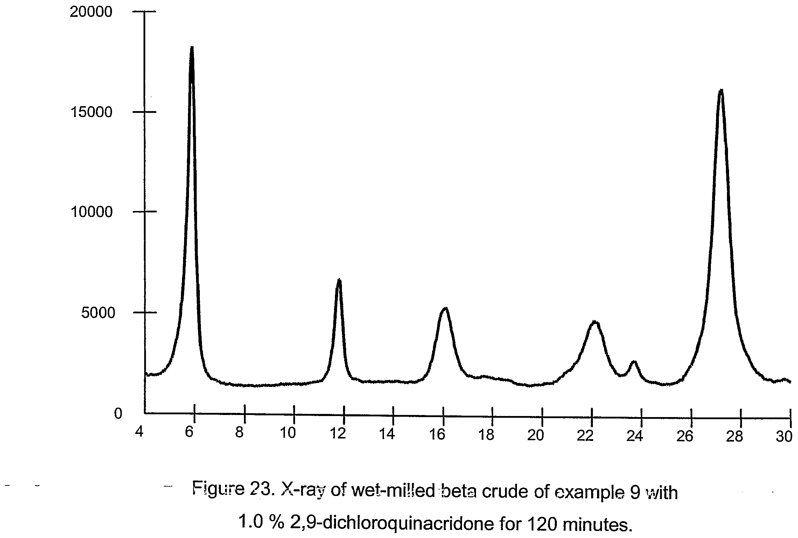

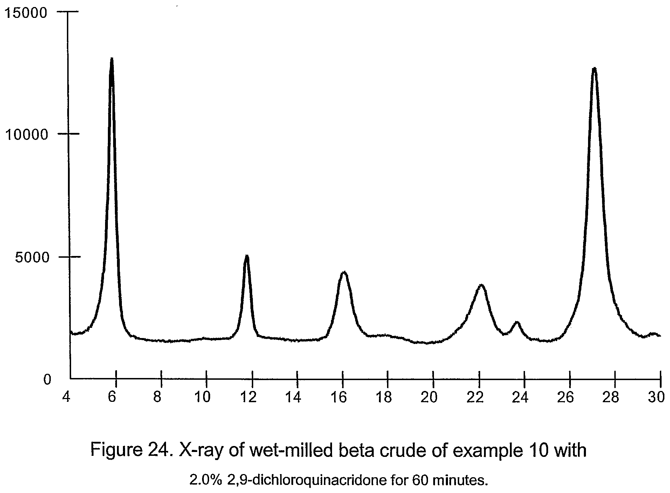

### Example B-3 is repeated for examples B-6 to B-11 except; milling time is varied, amount of 2,9-dichloroquinacridone is also varied and no Staybelite Ester® is added. See figures 20-24.

X-rays of the milled products shows no peak at 14θ. Thus 2,9-dichloroquinacridone functions as an alpha phase inhibitor at varying dosage levels and varying milling times.

**Table 1**

| Example (beta-crude milling) | Milling time in minutes | 2,9-dichloroquinacridone in wt. % | Method of Milling |
|---|---|---|---|
| B-6 | 60 | 0.5 | Wet-milling |
| B-7 | 120 | 0.5 | Wet-milling |
| B-8 | 60 | 1.0 | Wet-milling |
| B-9 | 120 | 1.0 | Wet-milling |
| B-10 | 60 | 2.0 | Wet-milling |
| B-11 | 120 | 2.0 | Wet-milling |

### Gamma-Quinacridone

### EXAMPLE B-12

To a dispersion mill 100 lbs Cylpebs® and 10 lbs of spikes is charged followed by a charge of 1532.2 grams aluminum sulfate (anhydrous), 647.1 grams crude gamma quinacridone, 20.4 grams 4,11-dichloroquinacridone, 13.6 grams 2,9-dichloroquinacridone, and 18.7 grams dimethylglutarate. Grinding continues at 40 rpm for 5 hours.

The mill powder is extracted with sulfuric acid and water (1.5% sulfuric acid) with stirring for 2 hr at 90 °C. Water is then added to cool the extraction mixture to about 60 to about 65 °C, with filtering and washing to neutral pH. The finished gamma-quinacridone is dried to less than 1 % moisture before formulation with alkyl melamine paint and testing to determine color space in Table 5.

When crude gamma-quinacridone is dispersion milled with 2,9-dichloroquinacridone, then extracted as above, the gamma phase is preserved. See color space in Table 5 for example B-12.

### EXAMPLES B-13 to B-18

### Crude gamma-quinacridone aqueous milling with 2,9-dichloroquinacridone

Example B-3 is repeated in examples B-13 to B-18 except no Staybelite Ester® was added and the crude pigment milled is gamma-quinacridone. The milling times and amounts of 2,9 dichloroquinacridone added during wet-milling are varied as in Table 2 below. Table 5 below shows color data.

**Table 2**

| Example | Milling time in minutes | 2,9-dichloroquinacridone in wt. % | Method of Milling |
|---|---|---|---|
| B-12 | 120 | 2.0 | Dispersion milling and extraction |
| B-1 3 | 60 | 0.5 | Wet-milling |
| B-14 | 90 | 0.5 | Wet-milling |
| B-15 | 120 | 1.0 | Wet-milling |
| B-16 | 120 | 0.5 | Wet-milling |
| B-17 | 120 | 3.0 | Wet-milling |
| B-1 8 | 30 | 0.5 | Wet-milling |

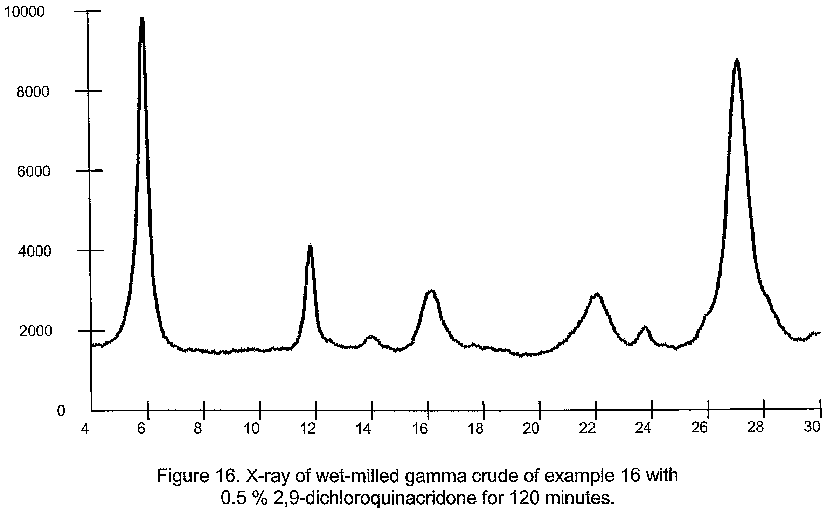

Milling with 2,9-dichloroquinacriodone directs the gamma-quinacridone to beta and/or alpha, depending on the amount of 2,9-dichloroquinacridone. When the gamma crude is milled with 0.5% 2,9-dichloroquinacridone for a maximum of 2 hours the gamma is converted to beta crude predominantly, See X-ray Figure 16. As the amount of 2,9-dichloroquinacridone is increased from 1.0% to 3% 2,9-dichloroquinacridone, the amount of alpha conversion increases. Thus the gamma crude milling in the presence of 2,9-dichloroquinacridone directs the crude gamma predominantly to beta when dosages of approximately 0.5 wt. % or less of 2,9-dichloroquinacidone are used as an additive. Predominantly means for the purposes of the invention, that greater than about 85 % of the gamma crude is converted to beta, preferably about 90% of the gamma crude is converted to beta phase.

Comparison examples are prepared in order to examine X-ray pattern of alpha-quinacridone and beta-quinacridone mixture. Samples are generated by physical mixing the known beta-quinacridone and alpha-quinacridone in a given percentage, X-ray spectra are obtained.

### COMPARISON EXAMPLE B-1

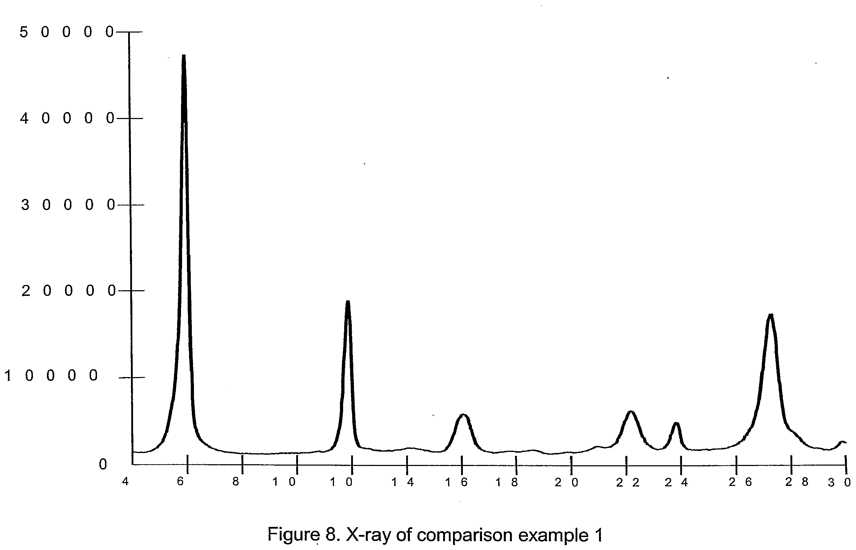

To a 100mL solid sample bottle is added 0.250g of alpha-quinacridone obtained from milling process, and 2.0g of beta-quinacridone crude, alpha-quinacridone and beta-quinacridone weight ratio equals 1.0/8.0. Above sample is placed in a shaker and mixes for 60 minutes before submitting for X-ray. A peak at 14θ is corresponding to alpha-quinacridone. X-ray spectrum is shown in Figure 8.

### COMPARISON EXAMPLE B-2

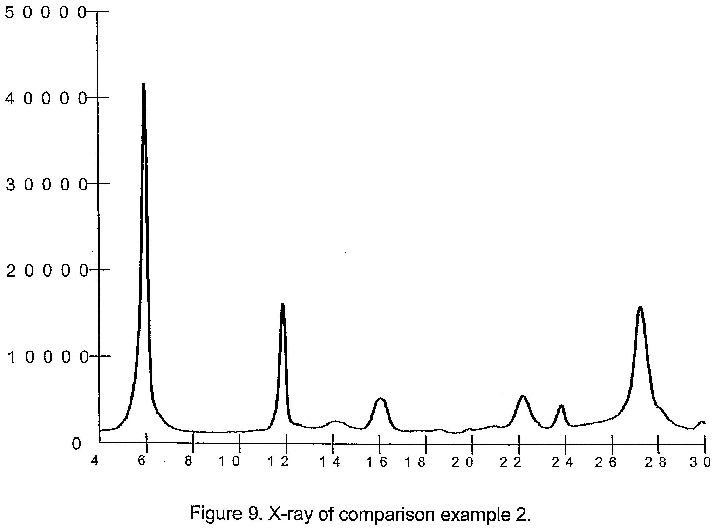

To a 100mL solid sample bottle is added 0.600g of alpha-quinacridone obtained from milling process, and 2.0g of beta-quinacridone crude, alpha-quinacridone and beta-quinacridone weight ratio equals 1.0/3.3. Above sample is placed in a shaker and mixes for 60 minutes before submitting for X-ray. A peak at 14θ corresponds to alpha-quinacridone. X-ray spectrum is shown in Figure 9.

### COMPARISON EXAMPLE B-3

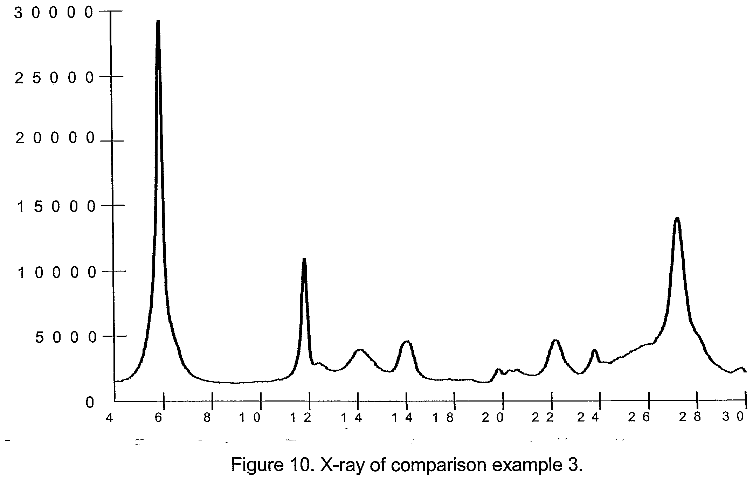

To a 100mL solid sample bottle is added 1.0g of alpha-quinacridone obtained from milling process, and 1.0 g of beta-quinacridone crude, alpha-quinacridone and beta-quinacridone weight ratio equals 1/1. Above sample is placed in a shaker and mixes for 60 minutes before submitting for X-ray. A peak at 14θ corresponding to alpha-quinacridone. X-ray spectrum is shown in Figure 10.

### Color of Aqueous Milled Beta-Quinacridone vs. Crude in Alkyd/Melamine Paint ^{a}

**Table 3**

| Sample | Tint | | Color | | | Masstone | | Color | |
|---|---|---|---|---|---|---|---|---|---|
| | L | C | H | Str. | | L | C | H | Trans. |
| Crude β-quinacridone | 69.9 | 18.6 | 337.5 | 100.0 | | 38.5 | 44.3 | 9.7 | STD |
| Example B-1 | 62.0 | 28.5 | 326.7 | 224.9 | | 35.0 | 40.5 | 6.2 | + 3.7 |
| Example B-2 | 61.3 | 27 | 322.2 | 353.9 | | 34.3 | 36.8 | 9.0 | |
| Example B-3 | 62.2 | 25.3 | 324.6 | 354.9 | | 34.7 | 37.6 | 9.3 | |
| Example B-4 | 62.1 | 25.1 | 323.9 | 357.9 | | 34.5 | 37.0 | 8.8 | |
| Example B-5 | 61.7 | 25.4 | 323.3 | 357.9 | | 34.4 | 36.6 | 8.8 | |

**Table 4**

| Sample | Tint | | Color | | | Masstone | | Color | |
|---|---|---|---|---|---|---|---|---|---|
| | L | C | H | Str. | | L | C | H | Trans. |
| Commercial beta-Violet | 60.4 | 28.5 | 321.9 | 100.0 | | 33.6 | 34.2 | 8.9 | STD |
| Example B-6 | 61.4 | 27.0 | 326.7 | 92.0 | | 35.6 | 36.2 | 9.1 | -7.3 |
| Example B-7 | 61.6 | 25.9 | 324.9 | 90.0 | | 35.2 | 37.0 | 8.6 | -6.5 |
| Example B-8 | 61.4 | 27.3 | 325.4 | 94.0 | | 35.4 | 37.9 | 8.3 | -5.6 |
| Example B-9 | 61.6 | 26.5 | 323.9 | 92.0 | | 35.1 | 37.2 | 7.8 | -4.9 |
| Example B-10 | 61.2 | 27.6 | 325.6 | 94.0 | | 35.3 | 38.1.6 | 8.7 | -5.4 |
| Example B-11 | 61.3 | 26.4 | 323.3 | 93.0 | | 34.8 | 36.6 | 8.4 | -4.4 |

### Color of Aqueous Milled gamma-quinacridone vs. Standard gamma-quinacridone in Alkyd/Melamine Paint ^{a}

**Table 5**

| Example | Tint | | Color | | | Masstone | | Color | |
|---|---|---|---|---|---|---|---|---|---|
| | L | C | H | Str, | | L | C | h | Trans. |
| Commercial gamma Red | 62.5 | 46.2 | 352.9 | 100.0 | | 41.2 | 53.0 | 21.8 | STD |
| Example B-12(dispersion milling and extraction) | 62.6 | 45.5 | 353.1 | 99 | | 41.8 | 54.5 | 22.0 | -0.8 |
| Example B-13 | 62.9 | 46.2 | 354.5 | 104.0 | | 42.2 | 54.5 | 22.2 | -7.6 |
| Example B-14 | 63.4 | 47.4 | 355.4 | 103.0 | | 43.0 | 55.2 | 22.7 | -2.1 |
| Example B-15 | 60.3 | 41.6 | 345.8 | 105.0 | | 36.6 | 43.2 | 14.8 | 3.5 |
| Example B-16 | 55.3 | 28.6 | 326.7 | 154.0 | | 32.6 | 28.4 | 4.9 | 1.2 |

### a. Alkyl Melamine Paint Formulation:

### Millbase:

To a 8 oz jar is added 1.2g of pigment sample, 0.6g of disperbyk 161 (high molecular weight block co-polymer dispersing additive, 30% active), 56.2g of alkyd melamine resin, 100g glass beads (Ø 2 mm). Above mixture is placed in a Skandex and milled for 2 hours. Pigment slurry is separated form glass beads, and collected as millbase. Pigment percentage is 2.1%.

### Masstone Color:

7.5g of above millbase is diluted with 22.5g of alkyd melamine resin, the pigment dispersion is drawn down on a Black/White Carton with 100µm wet film wired bar coater (No. 8) with the KCC automatic film applicator, dried 30 minutes @130°C.

### Tint Color:

16.7g of above millbase is diluted with 15.0g of white paint (20% TiO₂), the pigment dispersion is drawn down on a White Card with 100µm wet film wired bar coater (No. 8) with the KCC automatic film applicator, dried 30 minutes @130°C.

## Claims

1. A process for particle size reduction of organic pigments to improve the optical properties of pigments wherein the process comprises
(a) milling a mixture comprising:
i.) 10-60 wt. % one or more crude organic pigments,
ii.) 0.1-25 wt. % of a water-soluble styrene copolymer dispersant based on the dry weight of the crude organic pigment,
iii.) 0.1-1.0 wt. % of defoamer,
iv.) optionally 0.1-5.0 wt.% of an additive based on the dry weight of the crude organic pigment, and
v.) greater than 10 wt. % of water; wherein the weight percent of components i.), iii.) and v.) are based an the total weight of the mixture,
wherein by continuously circulating the mixture through a milling media a particle size range of 30 to 300 nm of the milled pigment or milled pigment mixture is obtained, and
(b) isolating the organic pigment.

2. The process according to claim 1 wherein the crude organic pigment is chosen from perylenes, quinacridones, phthalocyanines, isoindolines, dioxazines, triphendioxazines, 1,4-diketopyrrolopyrroles, anthrapyrimidines, anthranthrones, flavanthrones, indanthrones, perinones, pyranthrones, thioindigos, 4,4'-diamino-1,1-dianthraquinonyl, and azo compounds, as well as substituted derivatives or mixtures thereof; preferably the crude organic pigment is perylene, quinacridone, phthalocyanine, isoindoline, 1,4-diketopyrrolopyrroles and dioxazine pigments.

3. The process according to claim 1 or 2 wherein the crude organic pigment is a quinacridone of formula A or mixture of different derivatives of formula A 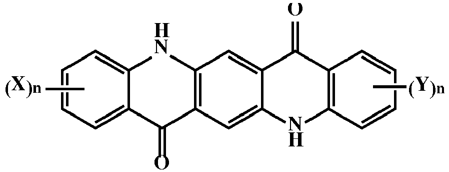 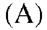 in which X and Y, independently of one another, are hydrogen, halogen, -OH, -NO₂, -CF₃, an C₁-C₄alkyl group, a substituted C₁-C₄alkyl group, a C₁-C₄alkoxy group, a substituted C₁-C₄alkoxy group, a phenyl group, a cyclohexyl group, a phenoxy group, -COOH, a -COO-C₁-C₄alkyl group, -SO₃H, a phenylamino group, a benzamino group, -N(CH₃)₂, -SO₂NH₂, -SO₂N(CH₃)₂, a pyridino group, -CONH₂ or -CON(CH₃)₂, especially H, F, Cl, Br, I, C₁-C₄alkyl, or C₁-C₄alkoxy, and n is 0, 1, or 2, especially 0, or 1.

4. The process according to claim 3 wherein the quinacridone is quinacridone, 2,9-dichloroquinacridone, 3,10-dichloroquinacridone, 4,11-dichloroquinacridone, 2,3,9,10-tetrachloroquinacridone, 2,4,9,11-tetrachloroquinacridone, 2,9-difluoroquinacridone, 2,9-dibromoquinacridone, 2,9-dimethylquinacridone, 3,10-dimethylquinacridone, 4,11-dimethylquinacridone, 2,4,9,11-tetramethylquinacridone, 2,9-di(tertbutyl)quinacridone, 2,9-dihydroxylquinacridone, 2,9-di(trifluoromethyl)quinacridone, 2,9-dimethoxyquinacridone, 2,9-diethoxyquinacridone, 2,4,9,11-tetramethoxyquinacridone, 2,9-dicarboxylquinacridone, 2,9-dichlorohexylquinacridone, 2,9-diphenylquinacridone, 2,9-di(dimethylamino)quinacridone, 2,9-di(dimethylaminosulfo)quinacridone, 2,9-di(dimethylaminocarbonyl)quinacridone, 3,10-dinitroquinacridone, 2,9-dimethyl-4,11-dichloroquinacridone, 2,9-dimethyl-4,11-dicarboxyquinacridone, and 2,9-dipyridinoquinacridone of beta or gamma crystal phase; preferably the quinacridone is an unsubstituted beta or gamma quinacridone or 2,9-dichloroquinacridone.

5. The process according to any of claims 1 to 4 wherein the styrene copolymer dispersant has a chemical structure shown in formula (B). 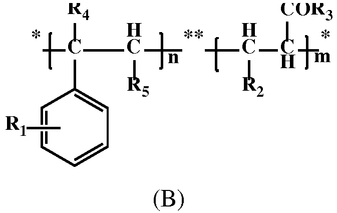 where R₁ is H, C₁-C₄ alkyl, fluoro, chloro, bromo, iodo, nitro, or amino groups, C₁-C₁₈ alkylene, or C₁-C₄ alkoxy groups; R₂ is H and C₁-C₄ alkyl group, R₃ is hydroxyl or C₁-C₄ alkoxy group, and 100 > n > 1, 100 > m > 1; R₄ and R₅ are independently of one another, H, or C₁-C₄ alkyl group.

6. The process according to any of claims 1 to 5 wherein the styrene copolymer dispersant of component (ii) has a ratio of styrene to (meth)acrylic monomer units that is equal to or greater than 2 to 1.

7. The process according to any of claims claim 1 to 6 wherein the styrene copolymer dispersant of component (ii) has a molecular weight range of 5,000 to 20,000.

8. The process according to any of claims claim 1 to 6 wherein the styrene copolymer dispersant of component (ii) has a molecular weight range of about 9,000 to 17,000.

9. The process according to any of claims 1 to 8 wherein the crude pigment or crude pigment mixture of component (i) has a particle size range of 0.3-4.0 µm.

10. The process according to any of claims 1 to 8 wherein the crude pigment or crude pigment mixture of component (i) has a particle size range of 1.0-3.0 µm.

11. The process according to any of claims 1 to 10 wherein the isolated milled pigment or milled pigment mixture has a particle size range of 30-300 nm.

12. The process according to any of claims 1 to 10 wherein the isolated milled pigment or milled pigment mixture has a particle size range of 40-200 nm.

13. A pigment composition prepared according to the process of any of claims 1 to 12.

14. A coating composition containing a pigment prepared according to the process of any of claims 1 to 12.

15. A composition for fiber and plastic applications containing a pigment prepared according to the process of any of claims 1 to 12.

16. A process for particle size reduction of beta- or gamma-quinacridone pigment crystals while maintaining the beta or gamma crystal phase to improve the optical properties of said pigments wherein the process comprises
(a) milling a mixture comprising:
i.) 10 to 60 wt. % of a composition comprising 2,9- dichloroquinacridone with beta or gamma-quinacridone wherein the wt. % of the 2,9-dichloroquinacridone is 0.1 % to 5.0 weight % based on the dry weight of the beta or gamma pigment,
ii.) 0.1-25 wt. % of a water-soluble styrene copolymer dispersant based on the dry weight of the composition comprising 2,9-dichloroquinacridone with beta or gamma-quinacridone,
iii.) 0.1-1.0 wt. % of defoamer,
iv.) optionally 0.1-5.0 wt. % of an additive based on the dry weight of the composition comprising 2,9-dichloroquinacridone with beta or gamma-quinacridone, and
v.) greater than 10 wt. % of water; wherein the weight percent of components i.), iii.) and v.) are based on the total weight of the mixture,
wherein the mixture is continuously circulated through a milling media, and
(b) isolating the beta- or gamma-quinacridone pigment crystals.

## Patentansprüche

1. Verfahren zum Verringern der Partikelgröße organischer Pigmente zum Verbessern der optischen Eigenschaften von Pigmenten, wobei das Verfahren umfasst:
(a) Mahlen eines Gemischs umfassend:
i.) 10-60 Gew.-% an einem oder mehreren rohen organischen Pigmenten,
ii.) 0,1-25 Gew.-% an einem wasserlöslichen Styrolcopolymer-Dispergiermittel bezogen auf das Trockengewicht des rohen organischen Pigments,
iii.) 0,1-1,0 Gew.-% Entschäumer,
iv.) gegebenenfalls 0,1-5,0 Gew.-% an einem Zusatzstoff bezogen auf das Trockengewicht des rohen organischen Pigments und
v.) mehr als 10 Gew.-% Wasser; wobei die Gewichtsprozentwerte der Komponenten i.), iii.) und v.) auf das Gesamtgewicht des Gemischs bezogen sind,
wobei durch kontinuierliches Zirkulieren des Gemischs durch ein Mahlmedium ein Partikelgrößenbereich des gemahlenen Pigments oder gemahlenen Pigmentgemischs von 30 bis 300 nm erhalten wird, und
(b) Isolieren des organischen Pigments.

2. Verfahren gemäß Anspruch 1, wobei das rohe organische Pigment ausgewählt ist aus Perylenen, Chinacridonen, Phthalocyaninen, Isoindolinen, Dioxazinen, Triphendioxazinen, 1,4-Diketopyrrolopyrrolen, Anthrapyrimidinen, Anthranthronen, Flavanthronen, Indanthronen, Perinonen, Pyranthronen, Thioindigos, 4,4'-Diamino-1,1-dianthrachinonyl und Azoverbindungen sowie substituierten Derivaten oder Gemischen davon; wobei das rohe organische Pigment vorzugsweise Perylen, Chinacridon, Phthalocyanin, Isoindolin, 1,4-Diketopyrrolopyrrol oder ein Dioxazin-Pigment ist.

3. Verfahren gemäß Anspruch 1 oder 2, wobei das rohe organische Pigment ein Chinacridon der Formel A oder ein Gemisch verschiedener Derivate der Formel A ist, 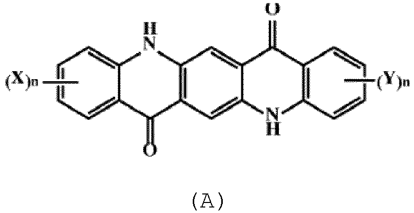 wobei X und Y unabhängig voneinander Wasserstoff, Halogen, -OH, -NO₂, -CF₃, eine C₁-C₄Alkylgruppe, eine substituierte C₁-C₄Alkylgruppe, eine C₁-C₄Alkoxygruppe, eine substituierte C₁-C₄Alkoxygruppe, eine Phenylgruppe, eine Cyclohexylgruppe, eine Phenoxygruppe, -COOH, eine -COO-C₁-C₄Alkylgruppe, -SO₃H, eine Phenylaminogruppe, eine Benzaminogruppe, -N(CH₃)₂, -SO₂NH₂, -SO₂N(CH₃)₂, eine Pyridinogruppe, -CONH₂ oder -CON(CH₃)₂ sind, insbesondere H, F, Cl, Br, I, C₁-C₄Alkyl oder C₁-C₄Alkoxy, und n 0, 1 oder 2 ist, insbesondere 0 oder 1.

4. Verfahren gemäß Anspruch 3, wobei das Chinacridon Chinacridon, 2,9-Dichlorchinacridon, 3,10-Dichlorchinacridon, 4,11-Dichlorchinacridon, 2,3,9,10-Tetrachlorchinacridon, 2,4,9,11-Tetrachlorchinacridon, 2,9-Difluorchinacridon, 2,9-Dibromchinacridon, 2,9-Dimethylchinacridon, 3,10-Dimethylchinacridon, 4,11-Dimethylchinacridon, 2,4,9,11-Tetramethylchinacridon, 2,9-Di(tert-butyl)chinacridon, 2,9-Dihydroxychinacridon, 2,9-Di(trifluormethyl)chinacridon, 2,9-Dimethoxychinacridon, 2,9-Diethoxychinacridon, 2,4,9,11-Tetramethoxychinacridon, 2,9-Dicarboxychinacridon, 2,9-Dichlorhexylchinacridon, 2,9-Diphenylchinacridon, 2,9-Di(dimethylamino)chinacridon, 2,9-Di(dimethylaminosulfo)chinacridon, 2,9-Di(dimethylaminocarbonyl)chinacridon, 3,10-Dinitrochinacridon, 2,9-Dimethyl-4,11-dichlorchinacridon, 2,9-Dimethyl-4,11-dicarboxychinacridon oder 2,9-Dipyridinochinacridon mit beta- oder gamma-Kristallphase ist; wobei das Chinacridon vorzugsweise ein unsubstituiertes beta- oder gamma-Chinacridon oder 2,9-Dichlorchinacridon ist.

5. Verfahren gemäß einem der Ansprüche 1 bis 4, wobei das Styrolcopolymer-Dispergiermittel eine in Formel (B) gezeigte chemische Struktur aufweist, 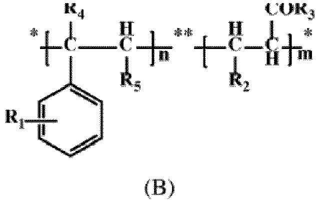 wobei R₁ H, C₁-C₄Alkyl, Fluor, Chlor, Brom, Iod, Nitro- oder Aminogruppe, C₁-C₁₈Alkylen- oder C₁-C₄Alkoxygruppe ist; R₂ H oder C₁-C₄Alkylgruppe ist; R₃ Hydroxy- oder C₁-C₄Alkoxygruppe ist und 100 > n > 1, 100 > m > 1; R₄ und R₅ unabhängig voneinander H oder C₁-C₄Alkylgruppe sind.

6. Verfahren gemäß einem der Ansprüche 1 bis 5, wobei das Styrolcopolymer-Dispergiermittel der Komponente (ii) ein Verhältnis von Styrol- zu (Meth)acryl-Monomereinheiten aufweist, das gleich oder größer als 2 zu 1 ist.

7. Verfahren gemäß einem der Ansprüche 1 bis 6, wobei das Styrolcopolymer-Dispergiermittel der Komponente (ii) einen Molekulargewichtsbereich von 5.000 bis 20.000 aufweist.

8. Verfahren gemäß einem der Ansprüche 1 bis 6, wobei das Styrolcopolymer-Dispergiermittel der Komponente (ii) einen Molekulargewichtsbereich von etwa 9.000 bis 17.000 aufweist.

9. Verfahren gemäß einem der Ansprüche 1 bis 8, wobei das rohe Pigment oder rohe Pigmentgemisch der Komponente (i) einen Partikelgrößenbereich von 0,3-4,0 µm aufweist.

10. Verfahren gemäß einem der Ansprüche 1 bis 8, wobei das rohe Pigment oder rohe Pigmentgemisch der Komponente (i) einen Partikelgrößenbereich von 1,0-3,0 µm aufweist.

11. Verfahren gemäß einem der Ansprüche 1 bis 10, wobei das isolierte gemahlene Pigment oder gemahlene Pigmentgemisch einen Partikelgrößenbereich von 30-300 nm aufweist.

12. Verfahren gemäß einem der Ansprüche 1 bis 10, wobei das isolierte gemahlene Pigment oder gemahlene Pigmentgemisch einen Partikelgrößenbereich von 40-200 nm aufweist.

13. Pigmentzusammensetzung, hergestellt gemäß dem Verfahren gemäß einem der Ansprüche 1 bis 12.

14. Beschichtungszusammensetzung, die ein Pigment enthält, das gemäß dem Verfahren gemäß einem der Ansprüche 1 bis 12 hergestellt ist.

15. Zusammensetzung für Faser- und Kunststoffanwendungen, die ein Pigment enthält, das gemäß dem Verfahren gemäß einem der Ansprüche 1 bis 12 hergestellt ist.

16. Verfahren zum Verringern der Partikelgröße von beta- oder gamma-Chinacridon-Pigmentkristallen unter Bewahrung der beta- oder gamma-Kristallphase zum Verbessern der optischen Eigenschaften der Pigmente, wobei das Verfahren umfasst:
(a) Mahlen eines Gemischs umfassend:
i.) 10 bis 60 Gew.-% an einer Zusammensetzung, die 2,9-Dichlorchinacridon mit beta- oder gamma-Chinacridon umfasst, wobei die Gew.-% des 2,9-Dichlorchinacridon 0,1 Gew.-% bis 5,0 Gew.-% bezogen auf das Trockengewicht des beta- oder gamma-Pigments betragen,
ii.) 0,1-25 Gew.-% an einem wasserlöslichen Styrolcopolymer-Dispergiermittel bezogen auf das Trockengewicht der Zusammensetzung, die 2,9-Dichlorchinacridon mit beta- oder gamma-Chinacridon umfasst,
iii.) 0,1-1,0 Gew.-% Entschäumer,
iv.) gegebenenfalls 0,1-5,0 Gew.-% an einem Zusatzstoff bezogen auf das Trockengewicht der Zusammensetzung, die 2,9-Dichlorchinacridon mit beta- oder gamma-Chinacridon umfasst, und
v.) mehr als 10 Gew.-% Wasser; wobei die Gewichtsprozentwerte der Komponenten i.), iii.) und v.) auf das Gesamtgewicht des Gemischs bezogen sind,
wobei das Gemisch kontinuierlich durch ein Mahlmedium zirkuliert wird, und
(b) Isolieren der beta- oder gamma-Chinacridon-Pigmentkristalle.

## Revendications

1. Procédé de réduction de la taille de particule de pigments organiques pour améliorer les propriétés optiques des pigments, le procédé comprenant :
(a) le broyage d'un mélange comprenant :
i.) 10 à 60 % en poids d'un ou plusieurs pigments organiques bruts,
ii.) 0,1 à 25 % en poids d'un dispersant qui est un copolymère de styrène hydrosoluble relativement au poids sec du pigment organique brut,
iii.) 0,1 à 1,0 % en poids d'antimousse,
iv.) optionnellement 0,1 à 5,0 % en poids d'un additif relativement au poids sec du pigment organique brut, et
v.) plus de 10 % en poids d'eau ; le pourcentage pondéral des constituants i.), iii.) et v.) étant exprimé relativement au poids total du mélange,
dans lequel, en faisant circuler en continu le mélange à travers un milieu de broyage, on obtient un intervalle granulométrique de 30 à 300 nm pour le pigment broyé ou le mélange de pigments broyés, et
(b) l'isolation du pigment organique.

2. Procédé selon la revendication 1, dans lequel le pigment organique brut est choisi parmi des pérylènes, des quinacridones, des phtalocyanines, des isoindolines, des dioxazines, des triphéndioxazines, des 1,4-dicétopyrrolopyrroles, des anthrapyrimidines, des anthranthrones, des flavanthrones, des indanthrones, des périnones, des pyranthrones, des thioindigos, le 4,4'-diamino-1,1-dianthraquinonyle et des composés azoïques, ainsi que des dérivés substitués ou des mélanges de ceux-ci ; le pigment organique brut étant préférablement un pigment pérylène, quinacridone, phtalocyanine, isoindoline, 1,4-dicétopyrrolopyrrole et dioxazine.

3. Procédé selon la revendication 1 ou 2, dans lequel le pigment organique brut est une quinacridone selon la formule A ou un mélange de différents dérivés selon la formule A 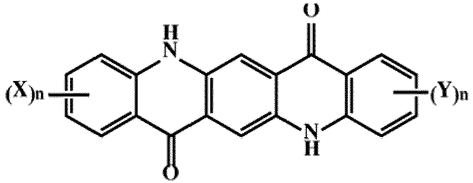 dans laquelle X et Y, indépendamment l'un de l'autre, sont un atome d'hydrogène, un atome d'halogène, un groupe -OH, -NO₂, CF₃, un groupe alkyle C₁-C₄, un groupe alkyle C₁-C₄ substitué, un groupe alcoxy C₁-C₄, un groupe alcoxy C₁-C₄ substitué, un groupe phényle, un groupe cyclohexyle, un groupe phénoxy, un groupe COOH, un groupe COO-alkyle C₁-C₄, un groupe -SO₃H, un groupe phénylamino, un groupe benzamino, un groupe N(CH₃)₂, -SO₂NH₂, -SO₂N(CH₃)₂, un groupe pyridino, un groupe -CONH₂ ou -CON(CH₃)₂, en particulier H, F, Cl, Br, I, alkyle C₁-C₄, ou alcoxy C₁-C₄, et n vaut 0, 1 ou 2, en particulier 0, ou 1.

4. Procédé selon la revendication 3, dans lequel la quinacridone est la quinacridone, la 2,9-dichloroquinacridone, la 3,10-dichloroquinacridone, la 4,11-dichloroquinacridone, la 2,3,9,10-tétrachloroquinacridone, la 2,4,9,11-tétrachloroquinacridone, la 2,9-difluoroquinacridone, la 2,9-dibromoquinacridone, la 2,9-diméthylquinacridone, la 3,10-diméthylquinacridone, la 4,11-diméthylquinacridone, la 2,4,9,11-tétraméthylquinacridone, la 2,9-di(tertbutyl)quinacridone, la 2,9-dihydroxylquinacridone, la 2,9-di(trifluorométhyl)quinacridone, la 2,9-diméthoxyquinacridone, la 2,9-diéthoxyquinacridone, la 2,4,9,11-tétraméthoxyquinacridone, la 2,9-dicarboxylquinacridone, la 2,9-dichlorohexylquinacridone, la 2,9-diphénylquinacridone, la 2,9-di(diméthylamino)quinacridone, la 2,9-di(diméthylaminosulfo)quinacridone, la 2,9-di(diméthylaminocarbonyl)quinacridone, la 3,10-dinitroquinacridone, la 2,9-diméthyl-4,11-dichloroquinacridone, la 2,9-diméthyl-4,11-dicarboxyquinacridone, et la 2,9-dipyridinoquinacridone de phase cristalline bêta ou gamma ; la quinacridone étant préférablement une quinacridone bêta ou gamma non substituée ou la 2,9-dichloroquinacridone.

5. Procédé selon l'une quelconque des revendications 1 à 4, dans lequel le dispersant qui est un copolymère de styrène a une structure chimique représentée dans la formule (B) 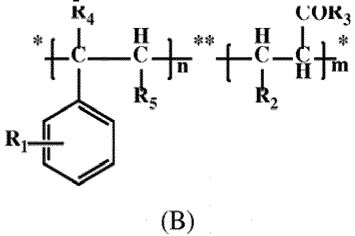 dans laquelle R₁ est H, des groupes alkyle C₁-C₄, fluoro, chloro, bromo, iodo, nitro, ou amino, des groupes alkylène C₁-C₁₈, ou alcoxy C₁-C₄ ; R₂ est H ou un groupe alkyle C₁-C₄, R₃ est un groupe hydroxyle ou alcoxy C₁-C₄, et 100 > n > 1, 100 > m > 1 ; R₄ et R₅ sont indépendamment l'un de l'autre H, ou un groupe alkyle C₁-C₄.

6. Procédé selon l'une quelconque des revendications 1 à 5, dans lequel le dispersant qui est un copolymère de styrène du constituant (ii) a un rapport des unités monomères de styrène contre les unités monomères (méth)acryliques qui est égal ou supérieur à 2 contre 1.

7. Procédé selon l'une quelconque des revendications 1 à 6, dans lequel le dispersant qui est un copolymère de styrène du constituant (ii) a une masse moléculaire dans la plage de 5 000 à 20 000.

8. Procédé selon l'une quelconque des revendications 1 à 6, dans lequel le dispersant qui est un copolymère de styrène du constituant (ii) a une masse moléculaire dans la plage d'environ 9 000 à 17 000.

9. Procédé selon l'une quelconque des revendications 1 à 8, dans lequel le pigment brut ou le mélange de pigments bruts du constituant (i) a un intervalle granulométrique de 0,3 à 4,0 pm.

10. Procédé selon l'une quelconque des revendications 1 à 8, dans lequel le pigment brut ou le mélange de pigments bruts du constituant (i) a un intervalle granulométrique de 1,0 à 3,0 pm.

11. Procédé selon l'une quelconque des revendications 1 à 10, dans lequel le pigment broyé isolé ou le mélange de pigments broyés a un intervalle granulométrique de 30 à 300 pm.

12. Procédé selon l'une quelconque des revendications 1 à 10, dans lequel le pigment broyé isolé ou le mélange de pigments broyés a un intervalle granulométrique de 40 à 200 pm.

13. Composition de pigment préparée par le procédé selon l'une quelconque des revendications 1 à 12.

14. Composition de revêtement contenant un pigment préparé par le procédé selon l'une quelconque des revendications 1 à 12.

15. Composition destinée à des applications dans les domaines des fibres et des matières plastiques, contenant un pigment préparé par le procédé selon l'une quelconque des revendications 1 à 12.

16. Procédé de réduction de la taille de particule de cristaux de pigment de type bêta-quinacridone ou gamma-quinacridone tout en conservant la phase cristalline bêta ou gamma pour améliorer les propriétés optiques desdits pigments, le procédé comprenant :
(a) le broyage d'un mélange comprenant :
i.) 10 à 60 % en poids d'une composition comprenant de la 2,9-dichloroquinacridone avec de la bêta-quinacridone ou de la gamma-quinacridone, le pourcentage pondéral de la 2,9-dichloroquinacridone étant de 0,1 % à 5,0 % en poids relativement au poids sec du pigment bêta ou gamma,
ii.) 0,1 à 25 % en poids d'un dispersant qui est un copolymère de styrène hydrosoluble relativement au poids sec de la composition comprenant de la 2,9-dichloroquinacridone avec de la bêta-quinacridone ou de la gamma-quinacridone,
iii.) 0,1 à 1,0 % en poids d'antimousse,
iv.) optionnellement 0,1 à 5,0 % en poids d'un additif relativement au poids sec de la composition comprenant de la 2,9-dichloroquinacridone avec de la bêta-quinacridone ou de la gamma-quinacridone, et
v.) plus de 10 % en poids d'eau ; le pourcentage pondéral des constituants i.), iii.) et v.) étant exprimé relativement au poids total du mélange,
dans lequel on fait circuler le mélange en continu à travers un milieu de broyage, et
(b) l'isolation des cristaux de pigment de type bêta-quinacridone ou gamma-quinacridone.
